# FASCICULE DE BREVET EUROPEEN

(11) **EP 3 387 130 B1**
(45) Date de publication et mention de la délivrance du brevet: **11.11.2020**
(21) Numéro de dépôt: 16825816.8
(22) Date de dépôt: 09.12.2016
(51) Int. Cl.: C12N 15/113

(54) **OUTILS DE THERAPIE GENIQUE EFFICACES POUR LE SAUT DE L'EXON 53 DE LA DYSTROPHINE**
EFFIZIENTE THERAPIE-WERKZEUGE ZUM SKIPPEN VON EXON 53 DES DYSTROPHIN-GENS
EFFICIENT GENE THERAPY TOOLS FOR EXON 53 SKIPPING OF THE DYSTROPHIN GENE

(30) Priorité: 09.12.2015 FR 1562036
(43) Date de publication de la demande: 17.10.2018
(73) Titulaire: Genethon, 91002 Evry (FR)
(72) Inventeur: PIETRI-ROUXEL, France, 92110 Clichy La Garenne (FR); FRANCOIS, Virginie, 44140 Remouille (FR)
(74) Mandataire: Cabinet Laurent & Charras
(86) Numéro de dépôt international: PCT/FR2016/053312
(87) Numéro de publication internationale: WO 2017/098187

(56) Documents cités:
- WO-A1-2011/057350
- WO-A2-2006/021724
- US-A1- 2014 315 977
- US-B2- 7 282 199
- POPPLEWELL L J ET AL: "Comparative analysis of antisense oligonucleotide sequences targeting exon 53 of the human DMD gene: Implications for future clinical trials", NEUROMUSCULAR DISORDERS, PERGAMON PRESS, GB, vol. 20, no. 2, 1 février 2010 (2010-02-01), pages 102-110, XP026878306, ISSN: 0960-8966 [extrait le 2010-01-15]

### SEQUENCE LISTING

<110> GENETHON
<120> Outils de thérapie génique efficaces pour le saut de l'exon 53 de la
   dystrophine
<130> G143-B-45963 PCT
<150> FR1562036
   <151> 2015-12-09
<160> 38
<170> BiSSAP 1.3.2
<210> 1
   <211> 212
   <212> DNA
   <213> Homo sapiens
<223> "Séquence de l'exon 53 du gène humain de la dystrophine"
<220>
   <223> Séquence de l'exon 53 du gène humain de la dystrophine
<400> 1
   ttgaaagaat tcagaatcag tgggatgaag tacaagaaca ccttcagaac cggaggcaac     60
   agttgaatga aatgttaaag gattcaacac aatggctgga agctaaggaa gaagctgagc    120
   aggtcttagg acaggccaga gccaagcttg agtcatggaa ggagggtccc tatacagtag    180
   atgcaatcca aaagaaaatc acagaaacca ag                                  212
<210> 2
   <211> 442
   <212> DNA
   <213> Artificial Sequence
<223> "U7-JR53"
<220>
   <223> U7-JR53
<400> 2
   taacaacata ggagctgtga ttggctgttt tcagccaatc agcactgact catttgcata      60
   gcctttacaa gcggtcacaa actcaagaaa cgagcggttt taatagtctt ttagaatatt     120
   gtttatcgaa ccgaataagg aactgtgctt tgtgattcac atatcagtgg aggggtgtgg     180
   aaatggcacc ttgatctcac cctcatcgaa agtggagttg atgtccttcc ctggctcgct     240
   acagacgcac ttccgcaaca ttcaactgtt gcctccggtt ctgaaggtgt tcttgtacaa     300
   tttttggagc aggttttctg acttcggtcg gaaaacccct cccaatttca ctggtctaca     360
   atgaaagcaa aacagttctc ttccccgctc cccggtgtgt gagaggggct ttgatccttc     420
   tctggtttcc taggaaacgc gt                                              442
<210> 3
   <211> 40
   <212> DNA
   <213> Artificial Sequence
<223> "JR53 (5902)"
<220>
   <223> JR53 (5902)
<400> 3
   cattcaactg ttgcctccgg ttctgaaggt gttcttgtac 40
<210> 4
   <211> 33
   <212> DNA
   <213> Artificial Sequence
<223> "N3 (5901)"
<220>
   <223> N3 (5901)
<400> 4
   caactgttgc ctccggttct gaaggtgttc ttg 33
<210> 5
   <211> 24
   <212> DNA
   <213> Artificial Sequence
<223> "N2 (5907)"
<220>
   <223> N2 (5907)
<400> 5
   ttgcctccgg ttctgaaggt gttc 24
<210> 6
   <211> 27
   <212> DNA
   <213> Artificial Sequence
<223> "N1 (5894)"
<220>
   <223> N1 (5894)
<400> 6
   ttgcctccgg ttctgaaggt gttcttg 27
<210> 7
   <211> 30
   <212> DNA
   <213> Artificial Sequence
<223> " Di/I (5903)"
<220>
   <223> Di/I (5903)
<400> 7
   caactgttgc ctccggttct gaaggtgttc 30
<210> 8
   <211> 36
   <212> DNA
   <213> Artificial Sequence
<223> "Dtex53 (5912)"
<220>
   <223> Dtex53 (5912)
<400> 8
   ttggctctgg cctgtcctct gttgcctccg gttctg 36
<210> 9
   <211> 19
   <212> DNA
   <213> Artificial Sequence
<223> "Scramble"
<220>
   <223> Scramble
<400> 9
   ggtgtattgc atgatatgt 19
<210> 10
   <211> 812
   <212> DNA
   <213> Artificial Sequence
<223> "ITR-U7-JR53"
<220>
   <223> ITR-U7-JR53
<400> 10
   ctgcgcgctc gctcgctcac tgaggccgcc cgggcaaagc ccgggcgtcg ggcgaccttt     60
   ggtcgcccgg cctcagtgag cgagcgagcg cgcagagagg gagtggccaa ctccatcact    120
   aggggttcct tgtagttaat gattaacccg ccatgctact tatctacgta gccatgctct    180
   agataacaac ataggagctg tgattggctg ttttcagcca atcagcactg actcatttgc    240
   atagccttta caagcggtca caaactcaag aaacgagcgg ttttaatagt cttttagaat    300
   attgtttatc gaaccgaata aggaactgtg ctttgtgatt cacatatcag tggaggggtg    360
   tggaaatggc accttgatct caccctcatc gaaagtggag ttgatgtcct tccctggctc    420
   gctacagacg cacttccgca acattcaact gttgcctccg gttctgaagg tgttcttgta    480
   caatttttgg agcaggtttt ctgacttcgg tcggaaaacc cctcccaatt tcactggtct    540
   acaatgaaag caaaacagtt ctcttccccg ctccccggtg tgtgagaggg gctttgatcc    600
   ttctctggtt tcctaggaaa cgcgttgtcg ctagagcatg gctacgtaga taagtagcat    660
   ggcgggttaa tcattaacta caaggaaccc ctagtgatgg agttggccac tccctctctg    720
   cgcgctcgct cgctcactga ggccgggcga ccaaaggtcg cccgacgccc gggctttgcc    780
   cgggcggcct cagtgagcga gcgagcgcgc ca                                  812
<210> 11
   <211> 18
   <212> DNA
   <213> Artificial Sequence
<223> "AAV18mers.R"
<220>
   <223> AAV18mers.R
<400> 11
   gtagataagt agcatggc 18
<210> 12
   <211> 22
   <212> DNA
   <213> Artificial Sequence
<223> "AAV22mers.F "
<220>
   <223> AAV22mers.F
<400> 12
   ctccatcact aggggttcct tg 22
<210> 13
   <211> 17
   <212> DNA
   <213> Artificial Sequence
<223> "AAV_M G B.P"
<220>
   <223> AAV_M G B.P
<400> 13
   tagttaatga ttaaccc 17
<210> 14
   <211> 22
   <212> DNA
   <213> Artificial Sequence
<223> "Alb.F"
<220>
   <223> Alb.F
<400> 14
   gctgtcatct cttgtgggct gt 22
<210> 15
   <211> 21
   <212> DNA
   <213> Artificial Sequence
<223> "Alb.R"
<220>
   <223> Alb.R
<400> 15
   actcatggga gctgctggtt c 21
<210> 16
   <211> 26
   <212> DNA
   <213> Artificial Sequence
<223> "AlbVIC.P "
<220>
   <223> AlbVIC.P
<400> 16
   cctgtcatgc ccacacaaat ctctcc 26
<210> 17
   <211> 20
   <212> DNA
   <213> Artificial Sequence
<223> "ex51 hDMD Fext"
<220>
   <223> ex51 hDMD Fext
<400> 17
   gttactctgg tgacacaacc 20
<210> 18
   <211> 20
   <212> DNA
   <213> Artificial Sequence
<223> "ex54 hDMD Rext "
<220>
   <223> ex54 hDMD Rext
<400> 18
   atgtggactt ttctggtatc 20
<210> 19
   <211> 20
   <212> DNA
   <213> Artificial Sequence
<223> "ex51 hDMD Fint "
<220>
   <223> ex51 hDMD Fint
<400> 19
   actagaaatg ccatcttcct 20
<210> 20
   <211> 20
   <212> DNA
   <213> Artificial Sequence
<223> "ex54 hDMD Rint "
<220>
   <223> ex54 hDMD Rint
<400> 20
   caagtcattt gccacatcta 20
<210> 21
   <211> 19
   <212> DNA
   <213> Artificial Sequence
<223> "Dys43-F "
<220>
   <223> Dys43-F
<400> 21
   cctgtggaaa gggtgaagc 19
<210> 22
   <211> 21
   <212> DNA
   <213> Artificial Sequence
<223> "Dys44-F "
<220>
   <223> Dys44-F
<400> 22
   cgatttgaca gatctgttga g 21
<210> 23
   <211> 20
   <212> DNA
   <213> Artificial Sequence
<223> "Dys56-R "
<220>
   <223> Dys56-R
<400> 23
   tgagagactt tttccgaagt 20
<210> 24
   <211> 20
   <212> DNA
   <213> Artificial Sequence
<223> "Dys57-R"
<220>
   <223> Dys57-R
<400> 24
   aagttcctgc agagaaaggt 20
<210> 25
   <211> 23
   <212> DNA
   <213> Artificial Sequence
<223> "hDys-44/54-F "
<220>
   <223> hDys-44/54-F
<400> 25
   cctgagaatt gggaacatgc taa 23
<210> 26
   <211> 18
   <212> DNA
   <213> Artificial Sequence
<223> "hDys-44/54-R "
<220>
   <223> hDys-44/54-R
<400> 26
   gccactggcg gaggtctt 18
<210> 27
   <211> 19
   <212> DNA
   <213> Artificial Sequence
<223> "hDys-44/54-P "
<220>
   <223> hDys-44/54-P
<400> 27
   ggtatcttaa gcagttggc 19
<210> 28
   <211> 24
   <212> DNA
   <213> Artificial Sequence
<223> "hDys-4/5-F "
<220>
   <223> hDys-4/5-F
<400> 28
   catgccctga acaatgtcaa caag 24
<210> 29
   <211> 25
   <212> DNA
   <213> Artificial Sequence
<223> "hDys-4/5-R "
<220>
   <223> hDys-4/5-R
<400> 29
   tccatctacg atgtcagtac ttcca 25
<210> 30
   <211> 24
   <212> DNA
   <213> Artificial Sequence
<223> "hDys-4/5-P "
<220>
   <223> hDys-4/5-P
<400> 30
   ttgcagaaca ataatgttga ttta 24
<210> 31
   <211> 20
   <212> DNA
   <213> Artificial Sequence
<223> "Dys49-F "
<220>
   <223> Dys49-F
<400> 31
   caaccggatg tggaagagat 20
<210> 32
   <211> 20
   <212> DNA
   <213> Artificial Sequence
<223> "Dys50-F"
<220>
   <223> Dys50-F
<400> 32
   ctctgagtgg aaggcggtaa 20
<210> 33
   <211> 20
   <212> DNA
   <213> Artificial Sequence
<223> "PO -F"
<220>
   <223> PO -F
<400> 33
   ggcgagctgg aagtgcaact 20
<210> 34
   <211> 20
   <212> DNA
   <213> Artificial Sequence
<223> "PO-R"
<220>
   <223> PO-R
<400> 34
   ccatcagcac cacagccttc 20
<210> 35
   <211> 24
   <212> DNA
   <213> Artificial Sequence
<223> "18S-F"
<220>
   <223> 18S-F
<400> 35
   tcaagaacga aagtcggagg ttcg 24
<210> 36
   <211> 23
   <212> DNA
   <213> Artificial Sequence
<223> "18S-R "
<220>
   <223> 18S-R
<400> 36
   ttatgctcaa tctcgggtgg ctg 23
<210> 37
   <211> 22
   <212> DNA
   <213> Artificial Sequence
<223> "Dys3-F "
<220>
   <223> Dys3-F
<400> 37
   gagaacctct tcagtgacct ac 22
<210> 38
   <211> 20
   <212> DNA
   <213> Artificial Sequence
<223> "Dys9-R "
<220>
   <223> Dys9-R
<400> 38
   gaggtggtga cataagcagc 20

## Description

### DOMAINE TECHNIQUE

La présente invention concerne des outils de thérapie génique particulièrement performants dans le traitement des maladies dystrophiques musculaires telles que la dystrophie musculaire de Duchenne (DMD).

Celle-ci repose sur le choix judicieux d'oligonucléotide antisens (AON) permettant le saut de l'exon 53 du gène de la dystrophine, véhiculés par un vecteur viral adéno-associé recombinant (AAVr), et avantageusement associés à un snRNA modifié.

### ETAT ANTERIEUR DE LA TECHNIQUE

La dystrophie musculaire de Duchenne (DMD) constitue la plus fréquente des maladies musculaires progressives dégénératives. Il s'agit d'une maladie génétique portée par le chromosome X qui touche environ 1 garçon sur 5 000. Elle résulte de mutations ou de délétions dans le gène de la dystrophine. La non-expression ou l'expression d'une dystrophine très anormale génère une fragilité de la fibre musculaire, ayant pour conséquence une destruction accélérée du tissu musculaire. Le déficit en dystrophine fonctionnelle cause une dégénérescence des fibres, une inflammation, une nécrose et un remplacement du muscle par du tissu adipeux, ce qui entraine une faiblesse musculaire progressive et une mort prématurée due à une déficience respiratoire ou cardiaque au cours des deuxième à quatrième décades de la vie (Moser, H., Hum Genet, 1984. 66(1): 17-40).

Le gène *dmd* (2.7 Mb), codant pour la protéine dystrophine, est composé de 79 exons et est situé au niveau du locus 21.2 du chromosome X. La dystrophine est une protéine modulaire présentant une région centrale composée de 24 domaines répétés de type "spectrin-like".

La majorité des mutations graves du gène de la dystrophine consiste en des délétions d'un ou plusieurs exons perturbant le cadre de lecture du messager final, des duplications d'une portion du gène, ou bien des mutations ponctuelles, présentes dans les régions codantes (ou exons), qui introduisent des codons stops ou des décalages de la phase de lecture.

Or, il a été remarqué que des formes tronquées de la dystrophine, en particulier dépourvues de certaines séquences répétées, sont parfaitement fonctionnelles ou du moins seulement partiellement défectives, comme par exemple dans la forme atténuée de la maladie appelée dystrophie musculaire de Becker (DMB).

Partant de cette constatation et de manière alternative à la thérapie génique classique consistant à apporter une copie intacte du gène défectueux (ce qui pose problème au vu de la taille de celui codant la dystrophine), différentes stratégies ont été envisagées pour tenter de "réparer" les dystrophines dysfonctionnelles.

Ainsi, le « saut d'exon » (« *exon skipping* » en anglais) est une approche thérapeutique qui consiste à administrer des oligonucléotides antisens (AON) complémentaires des séquences impliquées dans l'épissage des exons à masquer, afin de restaurer le cadre de lecture. En particulier, des sauts d'exons intervenant dans des régions de séquences répétées permettent d'obtenir une protéine dystrophine plus courte que la protéine native, mais qui est néanmoins fonctionnelle.

En pratique, le saut de l'exon 53 peut s'avérer bénéfique dans tous les cas où il permet de restaurer le cadre de lecture. Ceci vise théoriquement un nombre important de mutations pouvant être présentes dans le gène de la dystrophine, notamment des délétions de l'exon 52 (Δ52), des exons 50 à 52 (Δ50-52), des exons 49 à 52 (Δ49-52), des exons 48 à 52 (Δ48-52), des exons 47 à 52 (Δ47-52), des exons 45 à 52 (Δ45-52), des exons 43 à 52 (Δ43-52), des exons 42 à 52 (Δ42-52), des exons 41 à 52 (Δ41-52), des exons 40 à 52 (Δ40-52), des exons 39 à 52 (Δ39-52), des exons 38 à 52 (Δ38-52), des exons 37 à 52 (Δ37-52), des exons 36 à 52 (Δ36-52), des exons 35 à 52 (Δ35-52), des exons 34 à 52 (Δ34-52), des exons 33 à 52 (Δ33-52), des exons 32 à 52 (Δ32-52), des exons 31 à 52 (Δ31-52), des exons 30 à 52 (Δ30-52), des exons 29 à 52 (Δ29-52), des exons 28 à 52 (Δ28-52), des exons 27 à 52 (Δ27-52), des exons 26 à 52 (Δ26-52), des exons 25 à 52 (Δ25-52), des exons 24 à 52 (Δ24-52), des exons 23 à 52 (Δ23-52), des exons 21 à 52 (Δ21-52) et des exons 10 à 52 (Δ10-52), ou la duplication de l'exon 53.

Ainsi, cette stratégie permet notamment d'envisager de traiter différentes formes de DMD identifiées, en particulier celles associées à des délétions de l'exon 52 (Δ52), des exons 50 à 52 (Δ50-52), des exons 49 à 52 (Δ49-52), des exons 48 à 52 (Δ48-52), des exons 47 à 52 (Δ47-52), des exons 46 à 52 (Δ46-52), des exons 45 à 52 (Δ45-52), des exons 43 à 52 (Δ43-52) et des exons 10 à 52 (Δ10-52), ou à une duplication de l'exon 53.

Un des enjeux de cette technologie consiste à introduire de manière stable et durable un oligonucléotide antisens dans les fibres musculaires malades.

Il a été envisagé d'injecter directement lesdits AON, sous forme nue. Toutefois, au vu de la courte durée de vie de ces oligonucléotides dans le muscle, ce mode de traitement nécessite des injections régulières, relativement contraignantes. Des développements ont été réalisés pour modifier chimiquement ces oligonucléotides (morpholinos, 2'O méthyl, dérivés d'inosine, ...). Cette approche permet effectivement de les stabiliser *in vivo,* et ainsi d'améliorer leur efficacité et d'espacer leur injection. Toutefois et à forte dose, ce type d'oligonucléotides peut s'avérer toxique.

Alternativement, il a été tenté d'exprimer *in situ* ces séquences *via* des vecteurs d'expression permettant de les véhiculer dans les cellules cibles. Les vecteurs viraux recombinants adéno-associés (ou AAVr) se sont avérés particulièrement adaptés pour la transduction dans les muscles. Ces vecteurs permettent le transfert *in vivo* d'une cassette d'expression portant la séquence codant l'AON et conduisant à la synthèse continue de cet AON *in vivo.* Ainsi, l'injection de ces vecteurs a pour avantage de permettre une restauration de l'expression de la protéine à long terme. A titre d'exemple, Le Guiner et al. (Molecular Therapy, 2014, 22(11)1923-35) ont rapporté une efficacité notable du saut d'exon couplé à l'utilisation d'un AAV8 recombinant, et ce même 3,5 mois après une administration loco-régionale chez des chiens dystrophiques.

Dans ce cadre, l'utilisation de séquences issues de snRNA (pour « *small nucleotide RNA* ») dans ces vecteurs, dans lesquelles les séquences antisens sont insérées, a permis de limiter la dégradation des AONs *in vivo,* augmentant d'autant plus l'efficacité des traitements dans le temps. Cette stratégie a notamment été décrite dans la demande WO 2006/021724, rapportant l'utilisation de snRNA de type U7 (U7snRNA) pour transporter des AONs ciblant le gène de la dystrophine.

Les caractéristiques des snRNA en font des outils performants pour l'expression stable et nucléaire des séquences AON. En effet, les gènes correspondants sont de petite taille, exprimés de manière stable et continue au niveau nucléaire et ciblent les stades précoces de la transcription (pré-ARNm). En pratique et en rapport avec le U7snRNA, il s'agit d'insérer les séquences AON au niveau des sites de liaison aux protéines Sm des snRNA, de sorte qu'ils ne ciblent plus les ARN prémessagers des histones mais le ou les exons du gène d'intérêt ciblé, bloquant ainsi les sites consensus de l'épissage et permettant de corriger ou modifier la maturation de ce gène.

Il a, d'autre part, été suggéré d'ajouter, à ces séquences snRNA, des séquences dites « *exon splicing enhancer* », telles que, par exemple, des séquences permettant la liaison de facteurs impliqués dans l'épissage (en particulier la protéine hnRNPA1, pour « *heterogeneous* *ribonucleoprotein A1* »), afin d'améliorer encore les effets thérapeutiques observés (Goyenvalle et al., Mol Ther.; 17(7): 1234-1240, 2009).

Reste que le choix des AON, et donc des séquences codant ces AON dans le cas de vecteurs, est critique et s'est révélé particulièrement complexe. En effet, il est difficile de prédire quelle séquence sera efficace. Selon les exons ciblés, les séquences efficaces semblent varier et ne pas concerner les mêmes sites. Ce phénomène rend le choix des séquences AON particulièrement délicat et laborieux, même en utilisant des algorithmes complexes (Echigoya et al., Plos One, March 27, 2015, DOI: 10.1371/journal.pone.0120058).

Ainsi et en rapport avec l'exon 53, de nombreux antisens ont été proposés, variant aussi bien au niveau de la région de l'exon ciblée, que de la taille de l'oligonucléotide en question. Des séquences antisens candidates ont par exemple été proposées dans les documents suivants : WO2004/083446, WO2006/000057, US2010/0168212, WO2011/057350, WO2012/029986, WO2014/100714, US2014/0315977, US 2014/315977, Popelwell et al. (Mol Ther. 2009 Mar; 17(3):554-61 ; Neuromuscul Disord. 2010 Feb; 20(2):102-10). Une efficacité variable de ces séquences, testées sous la forme d'oligonucléotides nus ou éventuellement modifiés, possiblement en combinaison, a été décrite dans ces documents.

Au vu des enjeux cliniques, il existe toujours le besoin de développer un outil optimisé visant le saut de l'exon 53 sur l'ARN messager codant la dystrophine, c'est-à-dire permettant un saut efficace dudit exon et un fort niveau d'expression de la protéine correspondante, tronquée mais potentiellement fonctionnelle.

### OBJET DE L'INVENTION

La présente invention a pour but de traiter ou d'améliorer les formes de la Dystrophie Musculaire de Duchenne (DMD), dans lesquelles le saut de l'exon 53 sur l'ARN messager codant la dystrophine permet d'obtenir une protéine tronquée mais au moins partiellement fonctionnelle.

La solution proposée repose sur des oligonucléotides antisens (AON) judicieusement choisis, aussi bien au niveau de la région de l'exon qu'il cible, que de leur taille. Les séquences correspondantes sont introduites dans un système d'expression performant, constitué d'un vecteur viral recombinant adéno-associé (AAVr) comprenant avantageusement un snRNA, de préférence de type U7 avantageusement modifié au niveau du site de fixation aux protéines Sm.

De tels oligonucléotides antisens vectorisés ont permis d'obtenir une efficacité, aussi bien au niveau de l'expression des transcrits dépourvus d'exon 53 que de la production de la dystrophine tronquée, encore jamais atteinte à la connaissance du Demandeur et constituent donc un outil clinique très prometteur.

### Définitions

Les définitions ci-dessous correspondent au sens généralement utilisé dans le contexte de l'invention et sont à prendre en compte, à moins qu'une autre définition ne soit explicitement indiquée.

Au sens de l'invention, les articles « un » et « une » sont utilisés pour se référer à une ou à plusieurs (par exemple à au moins une) unités de l'objet grammatical de l'article. A titre d'exemple, « un élément » désigne au moins un élément, c'est-à-dire un élément ou plus.

Les termes « environ » ou « approximativement », utilisés en référence à une valeur mesurable telle qu'une quantité, une durée, et autres valeurs analogues, doivent être compris comme englobant des incertitudes de mesure de ± 20% ou de ± 10%, préférentiellement de ± 5%, encore plus préférablement de ± 1%, et de manière particulièrement préférée de ± 0,1% de la valeur spécifiée.

Intervalles: tout au long de la présente description, les différentes caractéristiques de l'invention peuvent être présentées sous forme d'intervalle de valeurs. Il doit être compris que la description de valeurs sous forme d'intervalle a uniquement pour finalité de rendre la lecture plus simple et ne doit pas être interprétée comme une limitation rigide de la portée de l'invention. En conséquence, la description d'un intervalle de valeurs devrait être considérée comme divulguant spécifiquement tous les intervalles intermédiaires possibles ainsi que chacune des valeurs au sein de cet intervalle. Par exemple, la description d'un intervalle allant de 1 à 6 devrait être considéré comme décrivant spécifiquement chacun des intervalles qu'ils comprend, tels que les intervalles allant de 1 à 3, de 1 à 4, de 1 à 5, de 2 à 4, de 2 à 6, de 3 à 6, etc., ainsi que chacune des valeurs dans cet intervalle, par exemple, 1, 2, 2,7, 3, 4, 5, 5,3 et 6. Cette définition vaut indépendamment de la portée de l'intervalle.

Le terme « isolé » doit être compris dans le contexte de l'invention comme synonyme de retiré ou extrait de son environnement ou état naturel. Par exemple, un acide nucléique ou un peptide isolé est un acide nucléique ou un peptide extrait de l'environnement naturel dans lequel on le trouve habituellement, qu'il s'agisse d'une plante ou d'un animal vivant par exemple. Ainsi, un acide nucléique ou un peptide naturellement présent dans un animal vivant n'est pas un acide nucléique ou un peptide isolé au sens de l'invention, tandis que le même acide nucléique ou peptide, partiellement ou complètement séparé des autres éléments présents dans son contexte naturel, est quant à lui « isolé » au sens de l'invention. Un acide nucléique ou un peptide isolé peut exister sous une forme substantiellement purifiée, ou peut exister dans un environnement non natif tel que, par exemple, une cellule hôte.

Dans le contexte de l'invention, les abréviations suivantes sont utilisées pour les bases d'acide nucléique les plus courantes. « A» » se réfère à l'adénosine, « C » se réfère à la cytosine, « G » désigne la guanosine, « T » désigne la thymidine, et « U » se réfère à l'uridine.

Sauf indication contraire, au sens de l'invention, une « séquence de nucléotides codant pour une séquence d'acides aminés » désigne toutes les séquences nucléotidiques qui codent pour la séquence d'acides aminés, y compris les séquences de nucléotides dégénérées permettant d'obtenir ladite séquence d'acide aminés. La séquence nucléotidique qui code pour une protéine ou un ARN ou un ADNc peut éventuellement comprendre des introns.

Les termes « codant » ou « codant pour », « code » ou « code pour » se réfèrent à la propriété inhérente aux séquences spécifiques de nucléotides dans un polynucléotide, tel qu'un gène, un ADNc ou un ARNm, à servir de matrice pour la synthèse d'autres polymères et macromolécules dans des processus biologiques, ayant soit une séquence définie de nucléotides (par exemple ARNr, ARNt et de l'ARNm), ou une séquence définie d'acides aminés, et les propriétés biologiques qui en découlent. Ainsi, un gène code pour une protéine si la transcription et la traduction de l'ARNm correspondant à ce gène produisent la protéine dans une cellule ou un autre système biologique. Tant le brin codant, dont la séquence nucléotidique est identique à la séquence d'ARNm et qui est généralement décrite dans les listages de séquences et bases de données, que le brin non codant, utilisé comme matrice pour la transcription d'un gène ou de l'ADNc, peuvent être désignés comme codant pour la protéine ou un autre produit de ce gène ou ADNc.

Le terme « polynucléotide » tel qu'utilisé dans le contexte de l'invention est défini comme une chaîne de nucléotides. En outre, les acides nucléiques sont des polymères de nucléotides. Ainsi, les termes acides nucléiques et polynucléotides tels qu'utilisés dans le cadre de l'invention sont interchangeables. Il est bien connu dans le domaine de la biologie moléculaire et du génie génétique que les acides nucléiques sont des polynucléotides, qui peuvent être hydrolysés en monomères. Les nucléotides sous forme monomère peuvent être hydrolysés en nucléosides. Tel qu'utilisé dans le contexte de l'invention, le terme polynucléotide désigne, à titre non limitatif, tout type de molécules d'acides nucléiques, c'est-à-dire les molécules d'acides nucléiques pouvant être obtenues par tout moyen disponible dans la technique, y compris par des moyens recombinants, à savoir le clonage de séquences d'acides nucléiques à partir d'une bibliothèque recombinante ou le génome d'une cellule, en utilisant des technologies de clonage ordinaire telle la PCR, ou par synthèse.

Dans le cadre de l'invention, le terme « oligonucléotide » désigne un polynucléotide dont, de manière préférentielle, la taille n'excède pas 100 nucléotides (ou bases), voire 95, 80, 75, 70, 65, 60, 55 ou même 50 nucléotides (ou bases).

Au sens de l'invention, les termes « peptide », « polypeptide » et « protéine » sont utilisés de manière interchangeable et font référence à un composé constitué de résidus d'acides aminés liés de manière covalente par des liaisons peptidiques. Une protéine contient par définition au moins deux acides aminés, sans limitation quant au nombre maximum d'acides aminés. Les polypeptides comprennent indifféremment plusieurs peptides et/ou protéines, lesquels comprennent eux-mêmes deux acides aminés ou plus reliés les uns aux autres par des liaisons peptidiques. Tel qu'il est utilisé ici, le terme se réfère à la fois à des chaînes courtes, qui sont également couramment désignés dans la technique comme des peptides, des oligopeptides et oligomères par exemple, et à des chaînes plus longues, qui sont généralement désignés dans la technique comme des protéines, dont il existe de nombreux types. Les « polypeptides » comprennent, par exemple, des fragments biologiquement actifs, des polypeptides substantiellement homologues, des oligopeptides, des homodimères, des hétérodimères, des variants de polypeptides, des polypeptides modifiés, des dérivés, des analogues, des protéines de fusion, entre autres. Les polypeptides comprennent des peptides naturels, des peptides recombinants, des peptides synthétiques, ou une combinaison de ceux-ci.

Les termes « identique » ou « homologue » se réfère à la similarité de séquence ou l'identité de séquence entre deux polypeptides ou entre deux molécules d'acide nucléique. Quand une position dans chacune des deux séquences comparées est occupée par la même base ou sous-unité monomère d'acide aminé (par exemple, lorsqu'une position dans chacune des deux molécules d'ADN est occupée par une adénine), alors les molécules sont homologues ou identiques pour cette position. Le pourcentage d'identité entre deux séquences est fonction du nombre de positions correspondant partagées par les deux séquences, et correspond à ce nombre divisé par le nombre de positions comparées et multiplié par 100. Par exemple, si 6 sur 10 des positions dans deux séquences appariées sont identiques, alors les deux séquences sont identiques à 60%. En règle générale, la comparaison est effectuée en alignant les deux séquences de façon à donner une homologie/identité maximale.

Un « vecteur » au sens de l'invention est une construction moléculaire qui comprend un acide nucléique isolé et qui peut être utilisée pour délivrer l'acide nucléique isolé à l'intérieur d'une cellule. De nombreux vecteurs sont connus dans l'art y compris, mais sans s'y limiter, les polynucléotides linéaires, des polynucléotides associés à des composés ioniques ou amphiphiles, des plasmides et des virus. Ainsi, le terme « vecteur » désigne par exemple un plasmide à réplication autonome ou un virus. Le terme doit également être interprété comme comprenant des composés non plasmidiques ou non viraux qui facilitent le transfert d'acides nucléiques dans des cellules, tels que, par exemple, les composés de polylysine, des liposomes, et analogues. A titre d'exemple de vecteurs viraux, on citera notamment les vecteurs adénoviraux, les vecteurs viraux adéno-associés, et les vecteurs rétroviraux.

Les termes « vecteur d'expression » désignent un vecteur comprenant un polynucléotide recombinant, lequel comprend des séquences de contrôle d'expression liées de manière opérationnelle à une séquence nucléotidique à exprimer. Un vecteur d'expression comprend notamment des éléments d'expression agissant en cis; d'autres éléments pour l'expression pouvant être fournis par la cellule hôte ou par un système d'expression in vitro. Les vecteurs d'expression au sens de l'invention incluent tous ceux connus dans l'art, tels que des cosmides, des plasmides (par exemple nus ou contenus dans des liposomes) et des virus (par exemple les lentivirus, les rétrovirus, les adénovirus et les virus adéno-associés) qui incorporent le polynucléotide recombinant.

Le terme « promoteur » tel qu'utilisé ici est défini comme une séquence d'ADN reconnue par la machinerie de synthèse de la cellule, ou la machinerie de synthèse introduite, nécessaire pour initier la transcription spécifique d'une séquence de polynucléotides.

Au sens de l'invention, les termes « séquence promotrice/régulatrice » désignent une séquence d'acide nucléique, nécessaire pour l'expression du polynucléotide lié de manière opérationnelle à la séquence promotrice/régulatrice. Dans certains cas, cette séquence peut être la séquence de base du promoteur, tandis que dans d'autres cas cette séquence peut également comprendre une séquence activatrice et d'autres éléments régulateurs, utiles pour l'expression du polynucléotide. La séquence promotrice/régulatrice peut être, par exemple, une séquence permettant l'expression du polynucléotide qui soit spécifique d'un tissu, c'est-à-dire se produisant préférentiellement dans ce tissu.

Au sens de l'invention, un promoteur « constitutif » est une séquence nucléotidique qui, lorsqu'elle est liée de manière opérationnelle à un polynucléotide, conduit à une expression du polynucléotide dans la plupart ou toutes les conditions physiologiques de la cellule.

Au sens de l'invention, un promoteur « inductible » est une séquence de nucléotides qui, lorsqu'elle est liée de manière opérationnelle à un polynucléotide, conduit à une expression du polynucléotide uniquement quand un inducteur du promoteur est présent dans la cellule.

Un promoteur « spécifique d'un tissu » est une séquence nucléotidique qui, lorsqu'elle est liée de manière opérationnelle à un polynucléotide, conduit à une expression du polynucléotide dans une cellule préférentiellement si la cellule est une cellule du type de tissu correspondant au promoteur.

Au sens de l'invention, le terme « anormal » lorsqu'il est utilisé en référence à des organismes, des tissus, des cellules ou des composants de ceux-ci, se réfère à ces organismes, tissus, cellules ou composants de ceux-ci qui diffèrent d'au moins une caractéristique observable ou détectable (par exemple âge, traitement, moment de la journée, etc.) de la caractéristique attendue dans des organismes, des tissus, des cellules ou des composants correspondants dit « normaux ».

Les termes « patient », « sujet », « individu » et synonymes sont utilisés dans le contexte de l'invention de manière interchangeable et se réfèrent à un animal, de préférence un mammifère. Dans certains modes de réalisation non limitatifs, l'animal est un humain. Il peut également s'agir d'une souris, un rat, un porc, un chien ou un primate non humain (NHP), tel que le macaque.

Au sens de l'invention, une « maladie » ou « pathologie » est un état de santé d'un animal dans lequel l'homéostasie de celui-ci est altérée, et qui, si la maladie n'est pas traitée, continue à se détériorer. En revanche, au sens de l'invention, un « trouble » est un état de santé dans lequel l'animal est capable de maintenir son homéostasie, mais dans lequel l'état de santé de l'animal est moins favorable à ce qu'elle serait en l'absence du trouble. Sans traitement, un trouble n'entraine pas nécessairement une détérioration de l'état de santé de l'animal dans le temps.

Dans le contexte de l'invention, une maladie ou un trouble est « allégé » ou « amélioré » si la gravité d'un symptôme de la maladie ou du trouble, ou la fréquence à laquelle le symptôme est ressenti par le sujet, ou les deux, est réduite. Cela inclut également la disparition de la progression de la maladie, c'est-à-dire la cessation de la détérioration de l'état de santé. Une maladie ou un trouble est « guéri » si la gravité d'un symptôme de la maladie ou du trouble, ou la fréquence avec laquelle un tel symptôme est ressenti par le patient, ou les deux, sont éliminés.

Dans le contexte de l'invention, un traitement « thérapeutique » est un traitement administré à un sujet qui présente les symptômes d'une pathologie, dans le but de diminuer ou d'éliminer ces symptômes.

Dans le contexte de l'invention, le « traitement d'une maladie ou d'un trouble » désigne la réduction de la fréquence ou de la sévérité d'au moins un symptôme d'une maladie ou d'un trouble chez un sujet.

Au sens de l'invention, une « quantité efficace » d'un composé est la quantité du composé qui est suffisante pour obtenir un effet bénéfique pour le sujet auquel le composé est administré. L'expression « quantité thérapeutiquement efficace » se réfère à une quantité qui est suffisante ou efficace pour prévenir ou traiter (en d'autres termes retarder ou prévenir l'apparition, prévenir l'évolution, inhiber, réduire ou inverser) une maladie ou un trouble, y compris soulager les symptômes de cette maladie ou ce trouble.

### DECRIPTION DETAILLE DE L'INVENTION

Selon un premier aspect, la présente demande concerne un vecteur viral recombinant adéno-associé (AAVr) comprenant une séquence codant un oligonucléotide antisens (AON) dirigé contre un segment d'au moins 33 bases de la région +30 à +69 de l'exon 53 de l'ARN pré-messager (pré-ARNm) de la dystrophine.

Dans le cadre de la présente demande et comme détaillé ci-dessous, les termes « dirigé contre », « cible », « s'hybride avec » et « complémentaire à » sont utilisés de manière équivalente.

Le vecteur AAVr selon l'invention comprend typiquement 2 composantes :
- La séquence d'acide nucléique recombinante encapsidée, aussi appelée « génome de l'AAVr ». Cette séquence d'acide nucléique recombinante comprend la séquence codant l'AON, lequel AON produit l'effet thérapeutique, en l'occurrence le saut de l'exon 53 du pré-ARNm de la dystrophine, lorsqu'elle est exprimée dans la cellule/le tissu cible ;
- La capside virale qui permet le transfert de gène et un certain tropisme tissulaire.

Selon l'invention, le génome du vecteur AAVr comprend donc une séquence codant un oligonucléotide antisens ou AON. Cette séquence se présente avantageusement sous forme d'ADN, et permet l'expression d'un AON capable de s'hybrider avec le pré-ARNm cible, en l'occurrence au niveau de l'exon 53 du pré-ARNm de la dystrophine.

De manière caractéristique selon l'invention, l'oligonucléotide antisens permet le saut de l'exon 53 sur l'ARN messager de la dystrophine. Il doit être compris que le saut de l'exon 53 à l'aide de l'AON selon l'invention s'effectue au stade de l'épissage de l'ARN pré-messager de la dystrophine. De manière connue dans ce domaine technique, l'AON choisi cible et s'hybride avec des régions impliquées dans l'épissage. En présence d'un tel AON, les sites d'épissage « masqués » par l'AON ne sont pas reconnus par la machinerie cellulaire de sorte que l'exon ciblé n'est pas intégré dans l'ARN messager résultant et donc « sauté ».

Dans le cadre de l'invention, on entend par « ARN pré-messager de la dystrophine » ou « pré-ARNm de la dystrophine » le produit de transcription du gène codant la protéine dystrophine, tel qu'obtenu avant l'étape d'épissage.

La présente demande vise tout gène codant une dystrophine, ou tout organisme comprenant un tel gène, pour lequel le saut de l'exon 53 peut avoir un intérêt, à savoir la production d'une protéine plus courte mais au moins partiellement fonctionnelle. Selon un mode de réalisation privilégié, le gène visé est le gène humain de la dystrophine. Le gène humain de la dystrophine est bien documenté : il a une taille de 2.7 Mb et est composé de 79 exons. Sa séquence complète est disponible dans les bases de données, par exemple dans la base de données NCBI sous la référence Gene ID : 1756 ou la base de données Ensembl sous la référence ENSG00000198947.

Avantageusement et dans le cadre de l'invention, on entend par « ARN pré-messager de la dystrophine » le pré-ARNm de la dystrophine d'origine humaine, en d'autres termes le pré-ARNm de la dystrophine humaine.

Les séquences du pré-ARNm de la dystrophine humaine sont bien connues et sont notamment accessibles grâce aux références indiquées ci-dessus en relation avec le gène entier.

Plus précisément, la présente invention vise l'exon 53 du pré-ARNm de la dystrophine humaine. De manière connue, celui-ci est constitué de 212 bases ou nucléotides et présente la séquence SEQ ID NO : 1 suivante :

Dans le cadre de la présente demande, les régions décrites ci-après sont identifiées par la position des nucléotides en référence à la séquence de l'exon 53 du pré-ARNm de la dystrophine, c'est-à-dire à la séquence SEQ ID NO : 1.

Dans la suite de la description, la numérotation utilisée est donc basée sur la séquence SEQ ID NO : 1, étant entendu que l'AON a nécessairement et par définition une séquence complémentaire à celle de sa cible. En pratique, la séquence codant l'AON, laquelle est comprise dans le vecteur de l'invention, peut par exemple être une séquence correspondant à l'inverse complémentaire de la région ciblée dans l'exon 53 du pré-ARNm de la dystrophine. Toutefois, et comme cela sera détaillé ci-après, des AON ne s'hybridant qu'avec un fragment de la région ciblée, ou s'hybridant avec cette région malgré la présence de mésappariements, sont aussi susceptibles d'être efficaces. Il doit donc être compris que la séquence codant l'AON selon l'invention comprend ou consiste en une séquence présentant au moins un certain pourcentage d'identité avec la séquence complémentaire de la région ciblée telle que définie au sens de l'invention, ou avec la séquence complémentaire d'au moins un segment de cette région ciblée.

Selon une première caractéristique, l'AON selon l'invention cible l'intégralité de la région +30 à +69 de l'exon 53 du pré-ARNm de la dystrophine, à savoir la région de 40 bases de séquence :
g tacaagaaca ccttcagaac cggaggcaac agttgaatg

Selon la présente divulgation, l'AON est dirigé contre un segment d'au moins 33 bases (ou nucléotides) de ladite région. En d'autres termes, l'antisens s'hybride avec au moins 33 nucléotides consécutifs de cette région constituée de 40 nucléotides.

Dans le cadre de la présente demande, on entend qu'un oligonucléotide s'hybride avec une séquence cible lorsque ledit oligonucléotide et la séquence cible forment, dans des conditions de stringence déterminée, modérée ou avantageusement forte, une molécule de pré-ARNm double brin.

Les conditions de stringence dépendent classiquement de conditions expérimentales telles que la température, la force ionique, et la concentration en agents dénaturants (tels que le formamide) dans le milieu réactionnel. Ces conditions et leurs possibles variations sont bien connues de l'homme de l'art. A titre d'exemple de conditions de forte stringence, on peut citer notamment les hybridation et lavage réalisés avec des compositions comprenant du chlorure de sodium (0,015 M) et du citrate de sodium (0,0015 M), à 65-68 °C (Voir Sambrook, Fritsch & Maniatis, Molecular Cloning. A Laboratory Manual, 2ème Ed, Cold Spring Harbor Laboratory, NY 1989). Des conditions de stringence modérée peuvent correspondre à des hybridation et lavage réalisés dans ces mêmes compositions, mais à une température inférieure, typiquement comprise entre 50 et 65°C.

En d'autres termes, l'oligonucléotide doit présenter une identité avec la séquence cible suffisante pour permettre un appariement des deux brins. Ceci est assuré dès lors que les deux séquences sont identiques, mais peut également avoir lieu en présence d'un ou plusieurs (en particulier 2, 3, 4 ou 5) nucléotides divergents, avantageusement lorsque ceux-ci sont situés à l'intérieur de la séquence. En termes d'identité, l'oligonucléotide présente avantageusement au moins 70%, 75%, 80%, 85% voire 90%, 91%, 92%, 93%, 94%, 95%, 96%, 97%, 98%, 99%, encore plus avantageusement 100% d'identité avec la région visée. Ainsi, de préférence, la séquence codant l'AON selon la présente demande ou ledit AON comprend ou consiste en une séquence présentant au moins 70%, 75%, 80%, 85% voire 90%, 91%, 92%, 93%, 94%, 95%, 96%, 97%, 98%, 99% d'identité avec la séquence complémentaire de la région allant des nucléotides +30 à +69 de la séquence SEQ ID NO : 1. Selon l'invention, la séquence codant l'AON consiste en une séquence présentant 100% d'identité avec la séquence complémentaire de la région allant des nucléotides +30 à +69 de la séquence SEQ ID NO : 1.

Selon la présente demande, l'AON est dirigé contre un segment de plus de 33 bases, à savoir 34, 35, 36, 37, 38, 39 ou même 40 bases de la région allant des nucléotides +30 à +69 de la séquence SEQ ID NO : 1. Selon l'invention, l'oligonucléotide s'hybride à l'intégralité de la région +30 à +69 de la séquence SEQ ID NO : 1. Le terme « s'hybride » s'entend comme défini ci-dessus, c'est-à-dire couvre le cas d'une identité parfaite entre l'oligonucléotide et la région cible, mais également les cas où il existe un ou plusieurs désappariements (appelés « mismatchs » en anglais). Ainsi, de préférence, la séquence codant l'AON selon l'invention consiste en une séquence complémentaire de la région allant des nucléotides +30 à +69 de la séquence SEQ ID NO : 1, ou en une séquence complémentaire d'un segment de 40 bases de la région allant des nucléotides +30 à +69 de la séquence SEQ ID NO : 1. Ainsi, de préférence, la séquence codant l'AON selon l'invention ou ledit AON consiste en une séquence présentant 100% d'identité avec la séquence complémentaire de la région visée, ou avec la séquence complémentaire d'un segment de 40 bases de la région allant des nucléotides +30 à +69 de la séquence SEQ ID NO : 1.

Selon la présente demande, l'AON peut être dirigé contre une région s'étendant de part et d'autre de la région visée. Ainsi, l'AON codé par ladite séquence peut s'hybrider avec une région s'étendant en 5' et/ou en 3' au-delà la région +30 à +69 de l'exon 53, à la condition avantageuse qu'une tel AON ait une efficacité, en termes de saut de l'exon 53, au moins égale à celle observé avec un AON dirigé contre un segment de plus de 33 bases de la région allant des nucléotides +30 à +69 de la séquence SEQ ID NO : 1, voire à celle d'un AON dirigé contre la région allant des nucléotides +30 à +69 de la séquence SEQ ID NO : 1.

Selon la présente divulgation, la taille de la séquence codant l'oligonucléotide antisens d'intérêt est au moins égale à la taille du segment visé dans l'exon 53, à savoir au moins 33 nucléotides.

Dans le cas où le segment visé a une taille supérieure, pouvant notamment varier de 34 à 40 nucléotides, la séquence codant l'AON selon la présente demande a une taille au moins égale, à savoir au moins 34, 35, 36, 37, 38, 39 voire même 40 bases. Selon un mode de réalisation particulier, la séquence codant l'AON selon la présente demande est constituée de 34 bases, préférentiellement 35, voire 36, 37, 38 ou 39 bases. Selon l'invention, la séquence codant l'AON selon l'invention est constituée de 40 nucléotides.

Selon un autre mode de réalisation particulier, la séquence codant l'AON mise en œuvre dans le cadre de la présente demande a une taille inférieure à 70 bases, voire inférieure ou égale à 65, 60, 55, 50 voire même 45, 44, 43, 42 ou 41 bases.

Selon l'invention, la séquence codant l'oligonucléotide antisens, comprise dans l'AAVr de l'invention, est constituée de la séquence SEQ ID NO : 3.

Selon un mode de réalisation particulier de la présente divulgation, la séquence codant l'oligonucléotide antisens comprise dans l'AAVr comprend ou est constituée de la séquence SEQ ID NO : 4.

Plus précisément :
- La séquence SEQ ID NO : 3 (laquelle est désignée dans les exemples « JR53 » ou « 5902 ») correspond à une séquence de 40 nucléotides codant un AON ciblant la région +30/+69 de l'exon 53 du pré-ARNm de la dystrophine ;
- La séquence SEQ ID NO : 4 (laquelle est désignée dans les exemples « N3 » ou « 5901 ») correspond à une séquence de 33 nucléotides codant un AON ciblant la région +33/+65 de l'exon 53 du pré-ARNm de la dystrophine.

Selon l'invention, ledit oligonucléotide est dirigé contre l'intégralité de la région +30 à +69 de l'exon 53 de l'ARN pré-messager du gène de la dystrophine. En conséquence, selon un mode de réalisation particulier, la séquence codant l'AON comprise dans l'AAVr de l'invention présente une taille de 40 bases et présente la séquence SEQ ID NO : 3.

De manière caractéristique selon l'invention, la séquence codant l'oligonucléotide antisens ou AON est portée ou véhiculée par un vecteur AAVr. En d'autres termes, la séquence codant l'oligonucléotide antisens ou AON est contenue ou comprise dans le génome de l'AAVr.

Les vecteurs viraux adéno-associés recombinants (AAVr) sont maintenant reconnus comme des outils performants pour le transfert de gènes, envisagés dans le traitement de nombreuses maladies. Les vecteurs AAVr présentent un certain nombre de caractéristiques qui les rendent adaptés à la thérapie génique, en particulier l'absence de pathogénicité, un pouvoir immunogène modéré et la capacité à transduire des cellules et tissus post-mitotiques de manière stable et efficace. L'expression d'un gène particulier véhiculé par un vecteur AAVr peut, en outre, être ciblée dans un ou plusieurs types cellulaires en choisissant de manière appropriée le sérotype de l'AAV, le promoteur et le mode d'administration.

Les AAVr sont issus de la modification par génie génétique des virus AAVs. Plus de 100 sérotypes naturels d'AAVs sont maintenant connus. Il existe de nombreux variants naturels au niveau de la capside de l'AAV, ce qui a permis la production et l'utilisation d'AAVr spécifiques avec des propriétés particulièrement adaptées pour les dystrophies musculaires. Des vecteurs AAVr peuvent être générés en utilisant des techniques classiques de biologie molécule, rendant possible l'optimisation des virus AAV pour la délivrance cellulaire spécifique de molécules d'acides nucléiques, pour minimiser l'immunogénicité, pour ajuster la stabilité et la durée de vie des particules, pour une dégradation efficace, et pour le transport au niveau du noyau. Comme mentionné ci-dessus, l'utilisation d'AAVr est un mode usuel pour délivrer des ADN exogènes en raison de leur non toxicité, du transfert efficace d'ADN et de la possibilité de les optimiser à des fins particulières.

Parmi les sérotypes d'AAVs isolés à partir d'humains ou de primates non humains et bien caractérisés, le sérotype 2 humain est le premier AAV qui a été utilisé pour la production de vecteur AAVr pour le transfert de gènes. D'autres sérotypes d'AAVs couramment utilisés dans la production de vecteurs AAVr incluent AAV1, AAV3, AAV4, AAV5, AAV6, AAV7, AAV8, AAV9, AAV10, AAV11 et AAV12.

Le génome viral des AAV est constitué d'environ 5000 nucléotides et comprend un gène codant pour les protéines de régulation et de réplication (*rep*) et un gène codant pour les protéines structurales (*cap*). Les séquences nécessaires en cis à la réplication du génome et à son empaquetage sont contenues dans un segment de 130 à 145 nucléotides retrouvé à chaque extrémité du génome (ITR pour « *inverted terminal repeat* »).

A partir de ces virus, un grand nombre de vecteurs recombinants et d'AAVr chimériques (hybrides) peuvent également être produits et utilisés.

Des fragments d'AAVs utiles pour l'assemblage en vecteurs incluent les protéines cap, en particulier vpl, vp2, vp3 et les régions hypervariables, les protéines rep, incluant rep 78, rep 68, rep 52 et rep 40, et les séquences codant ces protéines. Ces fragments peuvent être utilisés dans une grande variété de systèmes vectoriels et de cellules hôtes.

Dans le cadre de la production d'AAVr, ces fragments peuvent être utilisés seuls, en combinaison avec des séquences et fragments d'autres sérotypes d'AAVs, en combinaison avec des éléments d'autres AAVs ou avec des séquences virales non issues d'AAVs. Dans le cadre de l'invention, des AAVr appropriés incluent, sans limitation, des AAVr modifiés comprenant une protéine de capside non naturelle. Une telle capside artificielle peut être générée par toute technique adaptée, en utilisant une séquence d'AAV sélectionnée (par exemple un fragment de la protéine de capside vp1) en combinaison avec des séquences hétérologues qui peuvent être obtenues d'un sérotype sélectionné d'AAV différent, de portions non contiguës du même sérotype d'AAV, d'une source virale non AAV ou d'une source non virale. Un sérotype artificiel d'AAV peut être, sans limitation, une capside d'AAV chimérique, une capside d'AAV recombinante, ou une capside d'AAV « humanisée ». De tels exemples d'AAVs, ou des AAVs artificiels, incluent AAV2/8 (US 7,282,199), AAV2/5 (disponible auprès du National Institutes of Health), AAV2/9 (WO 2005/033321), AAV2/6 (US 6,156,303), et AAVrh8 (WO 2003/042397), parmi d'autres. Selon un mode de réalisation, les vecteurs utiles dans les compositions et méthodes de l'invention contiennent au minimum des séquences codant une capside d'un sérotype d'AAV sélectionné, par exemple une capside d'AAV8, ou un fragment de celle-ci. Selon un autre mode de réalisation, des vecteurs utiles contiennent au minimum des séquences codant une protéine rep d'un sérotype d'AAV sélectionné, par exemple une protéine rep AAV8, ou un fragment de celle-ci. Optionnellement, de tels vecteurs contiennent à la fois des protéines cap et rep d'AAV. Dans les vecteurs dans lesquels il y a à la fois rep et cap, les séquences des protéines rep et cap d'AAVs peuvent avoir pour origine le même AAV, par exemple toutes émanant d'un AAV8. De manière alternative, des vecteurs peuvent être utilisés dans lesquels les séquences rep émanent d'un sérotype d'AAV différent de celui dont émanent les séquences cap. Dans un mode de réalisation, les séquences rep et cap sont exprimées à partir de sources séparées (par exemple des vecteurs séparés, ou une cellule hôte et un vecteur). Dans un autre mode de réalisation, ces séquences rep sont fusionnées en phase aux séquences cap d'un AAV de sérotype différent pour former un vecteur AAVr chimérique tel que AAV2/8 (US 7,282,199).

A partir des AAVs, des génomes recombinants AAV peuvent être préparés, dans lesquels les gènes codant pour les protéines virales (*rep* et *cap*) sont délétés, seules restant les deux séquences répétées ITR. La production de vecteurs recombinants nécessite la multiplication et l'empaquetage de ces génomes recombinants. Cette étape est réalisée le plus souvent dans des cellules en culture que l'on transfecte à la fois avec le génome recombinant d'intérêt et des plasmides codant pour les protéines *rep* et *cap* manquantes (les protéines cap permettant la formation de la capside). Il est possible d'utiliser un génome recombinant et des gènes *rep* et *cap* provenant de sérotypes identiques ou différents. Un grand nombre de combinaisons sont ainsi possibles.

Dans le cas où les sérotypes sont identiques, on indiquera le seul sérotype correspondant au vecteur. Dans le cas où les sérotypes sont différents, on obtient des vecteurs AAVr dits hybrides pour lesquels on indique les sérotypes utilisés. A titre d'exemple, un vecteur AAVr de sérotype 2 est compris comme issu du seul sérotype 2. En revanche, un vecteur AAVr de sérotype 2/8 est un vecteur pour lequel on a utilisé un génome recombinant issu d'un AAV de sérotype 2, tandis que les gènes codant pour les protéines de la capside utilisés correspondent à ceux d'un AAV de sérotype 8.

Il a été montré que la capside des virus AAV, et en conséquence des vecteurs AAVr, est généralement associée à un tropisme cellulaire ou tissulaire particulier.

Selon l'invention, l'AAV mis en œuvre est avantageusement basé sur un AAV de sérotype 2 (AAV2), 8 (AAV8) ou 9 (AAV9). De manière adaptée, la capside est choisie pour permettre une transduction efficace voire préférentielle du muscle, qu'il soit squelettique, cardiaque ou lisse. Ainsi et préférentiellement, l'AAV mis en œuvre est avantageusement un AAV choisi dans le groupe suivant : AAV2/1, AAV2, AAV2/6, AAV2/8, AAV2/9, AAV2/10. De manière privilégiée, le vecteur est un vecteur AAV8, encore plus avantageusement un vecteur AAV2/8.

Concernant les vecteurs AAVr mis en œuvre dans le cadre de l'invention, le génome de l'AAV peut aussi bien être un acide nucléique simple brin (ss pour « *single stranded* »), double brin (ds pour « *double stranded* »), ou un acide nucléique auto-complémentaire (sc pour « *self complementary* »).

La production de vecteurs AAVr est bien connue de l'homme de l'art, et peut être réalisée par bi-transfection ou tri-transfection de cellules HEK 293, par le système viral herpès simplex ou à l'aide du système baculovirus. De manière avantageuse, les particules sont obtenues par bi- ou tritransfection de cellules HEK 293, ou à l'aide du système baculovirus.

Selon un mode de réalisation particulier, le vecteur selon l'invention contient ou comprend en outre une séquence codant un snRNA modifié. Plus précisément, la séquence codant l'oligonucléotide antisens (AON) selon l'invention est introduite ou insérée dans la séquence codant le snRNA. En d'autres termes et de préférence, le vecteur de l'invention comprend une séquence codant un oligonucléotide antisens, elle-même comprise dans une séquence codant un snRNA modifié. Ce mode de réalisation permet l'expression d'un oligonucléotide comprenant le snRNA modifié dans lequel est intégré l'AON. Comme déjà dit, l'intégration de l'AON au sein d'un snRNA modifié a pour effet de stabiliser l'expression de l'oligonucléotide qui en résulte, et en outre de permettre une expression nucléaire de ces oligonucléotides.

Les snRNA (pour « *small nuclear RNA* ») sont des ARN de petite taille, présents dans le noyau des cellules et impliqués dans certaines étapes de maturation des pré-ARN messagers. Ils sont appelés U1, U2, U3 ... jusqu'à U13. Il est bien connu que le snRNA natif comprend une région antisens spécifique du pré-ARN messager dont il assure la maturation. Cette séquence permet l'hybridation au pré-ARN messager cible. Par ailleurs, la région codant pour le snRNA natif (c'est-à-dire la séquence qui est effectivement transcrite) comprend notamment un domaine dit sm, qui code pour un site de fixation de la protéine Sm (pour « *small nuclear ribonucléoprotéine* »). Ce domaine est indispensable à l'activité de maturation des ARN pré-messager du snRNA (Grimm et al. EMBO J., 1993, 12(3): 1229-1238). La région codant pour le snRNA natif comprend en outre une séquence codant pour une forme tige-boucle dont il a été montré qu'elle stabilise l'interaction entre le snRNA et le pré-mRNA à maturer, facilitant ainsi l'epissage (Sharma et al., Genes Dev., 2014, 28(22): 2518-2531).

Dans le cadre de l'invention, on entend par « séquence codant un snRNA modifié », un oligonucléotide ayant pour séquence la séquence native du gène fonctionnel du snRNA, dans lequel les séquences impliquées dans la fonction initiale du snRNA sont inactivées et/ou modifiées, avantageusement par insertion de la séquence codant l'AON selon l'invention. Par « séquence native du gène fonctionnel », on entend au sens de l'invention la partie du gène endogène comprenant la région codant pour le snRNA (c'est-à-dire la séquence transcrite), ainsi que les régions régulatrices 5' et 3', et notamment le promoteur natif du snRNA.

De préférence, la séquence codant pour le snRNA modifié code un snRNA de type U7. Selon ce mode de réalisation, la séquence des sites de fixation des protéines Sm est modifiée de manière à inactiver la maturation des ARN pré-messagers codant les histones, avantageusement par insertion de la séquence codant l'AON selon l'invention.

Comme décrit en relation avec le snRNA de type U7, la séquence des sites de fixation des protéines Sm peut en outre être modifiée de manière à augmenter la concentration nucléaire en snRNA, par exemple remplacée par une séquence optimisée, avantageusement la séquence smOPT décrite par Schümperli et Pillai (Cell. Mol. Life Sci. 60: 2560-2570, 2004).

Par contre, et afin de faciliter l'épissage souhaité, la séquence codant pour la forme tige-boucle est préférentiellement native. En d'autres termes, elle est avantageusement non modifiée.

Comme déjà dit, le snRNA modifié mis en œuvre dans le cadre de l'invention est, de préférence, dépourvu de la séquence antisens spécifique native ciblant le pré-ARN messager visé par ce snRNA, en l'occurrence celui des histones pour le U7snRNA. Avantageusement, cette séquence est remplacée par une séquence codant pour l'oligonucléotide antisens d'intérêt, en l'occurrence ciblant l'intégralité de la région comprise entre les bases +30 et +69 de l'exon 53 du pré-ARNm de la dystrophine.

Au sens de l'invention, la séquence codant le snRNA modifié peut en outre être modifiée de manière à remplacer la séquence native du promoteur du snRNA par la séquence d'un autre promoteur, par exemple la séquence du promoteur d'un autre type de snRNA. A titre d'exemple et selon l'invention, une « séquence codant un snRNA modifié de type U7 » désigne une séquence codant un snRNA de type U7 ayant subi les modifications énoncées ci-dessus mais comprenant le promoteur natif du snRNA U7. Il est possible de remplacer ce promoteur par celui d'un autre snRNA, par exemple de type U1, U2 ou U3, ou par tout autre promoteur approprié pour la mise en œuvre de l'invention. Les différents promoteurs des snRNA sont bien connus et ont notamment été décrits par Hernadez (J Biol Chem.; 2001, 276(29):26733-6).

Dans le cadre de l'invention et parmi les différents types de snRNA, celui de type U7 (U7snRNA), normalement impliqué dans la maturation des ARN prémessagers codant les histones, est préférentiellement utilisé.

Dans ce cadre, il a en outre été montré qu'un domaine particulier, dit « kiss domaine » permet, lorsqu'il est fusionné à l'oligonucléotide antisens porté par le snRNA, de s'hybrider avec la forme tige-boucle du snRNA. Cette hybridation induit une modification de la structure secondaire du snRNA modifié qui améliore son interaction avec la machinerie moléculaire nécessaire à l'épissage. Ce domaine a été décrit notamment dans la demande WO 2011/113889, dont le contenu doit être considéré comme faisant partie de la présente demande. Ainsi, il est possible d'intégrer à la séquence codant le snRNA modifié selon l'invention une séquence codant pour le « kiss domaine » tel que décrit dans ce document.

En pratique, ces différentes modifications peuvent être introduites dans les séquences codant le snRNA au moyen de techniques usuelles en génie génétique, telle que la mutagénèse dirigée par PCR.

A noter que les séquences des snRNA sont hautement conservées entre les différentes espèces. Ainsi, la séquence codant le snRNA modifié utilisé dans le vecteur de l'invention peut être aussi bien d'origine humaine que murine. Préférentiellement, la séquence codant le snRNA modifié utilisé dans l'invention est d'origine murine.

Une construction typique selon l'invention, codant un snRNA dans lequel est insérée la séquence codant l'AON d'intérêt, va donc avantageusement comprendre :
- La région 5' non traduite du gène codant le U7 snRNA, comprenant notamment le promoteur, avantageusement d'origine murine, telle que par exemple illustré par les nucléotides 1 à 258 de la séquence SEQ ID NO : 2 ;
- La séquence codant l'AON dirigé contre l'intégralité de la région +30 à +69 de l'exon 53 du pré-ARNm de la dystrophine. A titre d'exemple les nucléotides 259 à 298 de la séquence SEQ ID NO : 2 illustrent une telle séquence, laquelle code un oligonucléotide de 40 bases (désignée JR53 dans les exemples) et de séquence SEQ ID NO : 3;
- La séquence de fixation de la protéine Sm modifiée, par exemple la séquence smOPT, correspondant aux nucléotides 299 à 309 de la séquence SEQ ID NO : 2 ;
- La séquence codant pour la forme tige-boucle, non modifiée et avantageusement d'origine murine, correspondant aux nucléotides 310 à 340 de la séquence SEQ ID NO : 2 ;
- La région 3' non traduite du U7 snRNA, avantageusement d'origine murine, telle que par exemple illustré par les nucléotides 341 à 442 de la séquence SEQ ID NO : 2.

De préférence, la séquence codant pour un oligonucléotide antisens d'intérêt est intégrée en amont de la séquence de fixation de la protéine Sm modifiée, à savoir en amont de la séquence smOPT.

Un snRNA modifié tel que décrit ci-dessus, avantageusement de type U7, et intégrant un oligonucléotide antisens d'intérêt au sens de l'invention, peut par exemple présenter la séquence SEQ ID NO : 2.

Il est à noter que d'autres séquences peuvent être intégrées dans la construction décrite ci-dessus. En particulier, d'autres séquences peuvent être intégrées dans le snRNA modifié portant la séquence codant l'AON d'intérêt, avantageusement en amont ou en aval de ladite séquence. Il peut en particulier être envisagé l'insertion d'au moins une séquence codant un autre AON d'intérêt, dirigé contre une autre région de l'exon 53 ou permettant le saut d'un autre exon de la dystrophine dont le saut présente également un intérêt.

La séquence codant le snRNA modifié, porteur de la séquence codant l'AON d'intérêt dans le cadre de l'invention est avantageusement insérée entre les 2 séquences ITR d'un AAV, par exemple de sérotype 2. Une construction correspondante est par exemple illustrée à la séquence SEQ ID NO : 10.

A noter qu'au vu de la taille des constructions envisagées dans le cadre de l'invention et de la capacité d'encapsidation des AAV, il est possible d'envisager l'intégration d'autres constructions entre les deux séquences ITR du vecteur AAVr selon l'invention :
- l'introduction d'une ou plusieurs copies supplémentaires (par exemple de 1 à 9, avantageusement de 1 à 3) de la séquence codant la construction selon l'invention (snRNA dans lequel est insérée la séquence codant l'AON d'intérêt), par exemple de séquence SEQ ID NO : 2 ;
- l'introduction d'une ou plusieurs autres séquences codant un snRNA modifié, porteur d'au moins une séquence codant un autre AON d'intérêt, dirigé contre une autre région de l'exon 53 ou permettant le saut d'un autre exon de la dystrophine dont le saut présente également un intérêt.

En d'autres termes et afin d'améliorer le saut d'exons, le vecteur AAVr selon l'invention peut comprendre plusieurs séquences codant pour des oligonucléotides antisens. Différents modes de réalisation sont envisageables, qui ne sont pas mutuellement exclusifs. Selon un premier mode de réalisation, le vecteur AAVr comprend plusieurs snRNA modifiés, chacun de ces snRNA comprenant une séquence codant pour un oligonucléotide antisens. Selon un deuxième mode de réalisation, le vecteur AAVr comprend un seul snRNA modifié, lequel comprend plusieurs séquences codant pour un ou plusieurs oligonucléotides antisens. Dans ce mode de réalisation, il est entendu que l'AAVr peut comprendre plusieurs séquences codant pour un même oligonucléotide antisens, et/ou des séquences codant pour des oligonucléotides antisens différents.

Fait également partie de l'invention tout type de cellule isolée, transduite par le vecteur AAVr décrit ci-dessus. Il s'agit avantageusement de cellules musculaires, en particulier de type fibres musculaires (myotubes) ou précurseurs musculaires (myoblastes). Selon un mode de réalisation particulier, les cellules embryonnaires humaines ayant requis la destruction d'un embryon humain sont exclues de la portée.

Un tissu musculaire isolé ou un organisme non humain transduit par ledit vecteur sont également compris dans l'étendue de la protection recherchée. Parmi les organismes non humains, les animaux sont préférés.

La présente demande décrit pour la première fois un potentiel thérapeutique pour le vecteur revendiqué. La présente invention se rapporte donc également à des compostions pharmaceutiques comprenant comme principe actif au moins un vecteur AAVr tel que défini dans la présente demande, ainsi que l'utilisation de ce vecteur comme médicament.

Selon un autre aspect, la présente invention concerne une composition, avantageusement une composition pharmaceutique ou médicament, comprenant un vecteur AAVr tel que décrit ci-dessus, et potentiellement d'autres molécules actives (d'autres produits de thérapie génique, des entités chimiques, des peptides, des protéines, ...), dédié au traitement de la même maladie ou d'une autre maladie.

Ainsi la présente invention offre des compositions pharmaceutiques comprenant un acide nucléique selon l'invention, en l'occurrence un vecteur AAVr selon l'invention. Une telle composition comprend une quantité thérapeutiquement efficace du vecteur selon l'invention et un véhicule pharmaceutiquement acceptable et inerte. Selon un mode de réalisation particulier, l'expression « pharmaceutiquement acceptable » signifie approuvé par une agence réglementaire fédérale ou d'un état, ou listé dans la pharmacopée américaine ou Européenne, ou toute autre pharmacopée reconnue comme admissible pour l'homme et l'animal. Le terme « véhicule » fait référence à un diluant, adjuvant, excipient ou support avec lequel l'agent thérapeutique est administré. De tels véhicules pharmaceutiques peuvent être des liquides, avantageusement stériles, tels que l'eau et les huiles, incluant ceux d'origine pétrolière, animale, végétale ou synthétique, tels que l'huile d'arachide, l'huile de soja, l'huile minérale, l'huile de sésame et autres. L'eau est un véhicule préféré lorsque la composition est administrée de manière intraveineuse. Des solutions salines, le dextrose aqueux, les solutions de glycérol peuvent également être utilisés comme véhicules liquides, en particulier dans le cas de solutions injectables. Des excipients pharmaceutiquement acceptable incluent l'amidon, le glucose, le lactose, le sucrose, le stéarate de sodium, le monostérate de glycérol, le talc, le chlorure de sodium, le glycérol, le propylène glycol, l'eau, l'éthanol et d'autres.

La composition, si nécessaire peut également contenir des quantités mineures d'agents mouillants ou émulsifiants, ou des agents tampon du pH. Ces compositions peuvent prendre la forme de solutions, suspensions, émulsions, formulations à libération prolongée, et autres. Des exemples de véhicules pharmaceutiquement acceptables sont par exemple décrits dans « Remington's Pharmaceutical Sciences » by E.W. Martin. De telles compositions contiennent une quantité thérapeutiquement efficace de l'actif, préférentiellement sous forme purifiée, avec une quantité adaptée de véhicule de sorte à obtenir la forme d'administration adaptée pour le patient.

Selon un mode de réalisation préféré, la composition est formulée en accord avec les procédures habituelles pour les compositions pharmaceutiques adaptées à l'administration intraveineuse chez l'homme. Classiquement, les compositions pour l'administration intraveineuse sont des solutions dans un tampon aqueux stérile isotonique. Si nécessaire, la composition peut également contenir un agent solubilisant et un anesthésique local tel que la lidocaïne pour soulager la douleur au site d'injection.

Selon un mode de réalisation, la composition selon l'invention est adaptée à l'administration chez l'homme. La composition est préférentiellement sous forme liquide, avantageusement sous la forme d'une composition saline, encore plus avantageusement sous la forme d'une composition contenant un tampon phosphate salin (PBS) ou une solution de type Ringer-Lactate.

La quantité de l'agent thérapeutique selon l'invention, en l'occurrence un vecteur AAV recombinant, qui est efficace pour le traitement des maladies dystrophiques peut être déterminée par des protocoles cliniques standards. De plus, des essais *in vitro* et/ou *in vivo* peuvent être mis en œuvre de manière optionnelle pour aider à la détermination des valeurs de dosages optimales. Le dosage précis à utiliser dans la composition peut également dépendre de la voie d'administration choisie, de la fréquence d'administration, du poids et/ou de l'âge du patient, de la gravité de la maladie et doit être décidé par le praticien au vu du contexte particulier de chaque patient.

Un mode d'administration adapté doit permettre la délivrance d'une quantité thérapeutiquement efficace de l'actif aux tissus cibles, en particulier aux muscles squelettiques et éventuellement au cœur et au diaphragme. Dans le contexte particulier de l'invention où l'actif est un vecteur AAV recombinant, la dose thérapeutique est définie en termes de quantité de particules virales (vg pour « *viral genome* ») administrée par kilogramme (kg) du patient.

De manière adaptée, la quantité adaptée doit permettre :
- Le saut de l'exon 53 pour au moins 10%, voire 20%, 30%, 40%, 50%, 60%, 70%, 80%, 90%, 95% voire même 100% des transcrits de la dystrophine. Ceci peut être évalué par toute technique connue de l'homme du métier, par exemple par RT-PCR;
- La production d'une dystrophine tronquée (au moins dépourvue de la partie codée par l'exon 53) représentant au moins 5%, voire 10%, 20%, 30%, 40%, 50%, 60%, 70%, 80%, 90%, 95% voire même 100% de la dystrophine normalement produite dans un organisme sain. Ceci peut être évalué par toute technique connue de l'homme du métier, par exemple par western blot.

En pratique, et notamment en cas d'administration systémique, la composition est avantageusement administrée à une dose inférieure ou égale à 10¹⁵ vg/kg, voire même 10¹⁴ vg/kg. Elle peut être comprise entre 10¹² vg/kg et 10¹⁴ vg/kg, de préférence comprise entre 2.10¹² vg/kg et 5.10¹³ vg/kg. De manière connue, une dose aussi faible que possible permettant d'obtenir un résultat satisfaisant est privilégiée afin notamment d'éviter les problèmes de toxicité ou de réactions immunitaires.

Les voies d'administration disponibles sont l'administration topique (locale), entérale (effet général mais délivrance via le tractus gastro-intestinal (GI)), ou parentale (action systémique mais délivrance par des voies autres que le tractus GI). La voie d'administration préférée de la composition selon l'invention est la voie parentale et inclut l'administration intramusculaire (dans le muscle) et l'administration systémique (dans le système circulant). Dans ce contexte, le terme «injection» (ou perfusion) comprend l'administration intravasculaire, en particulier intraveineuse (IV), et intramusculaire (IM). Les injections sont généralement réalisées à l'aide de seringue ou de cathéter.

Selon un mode de réalisation particulier, la délivrance systémique de la composition comprend l'administration locale de la composition au niveau d'un site de traitement, par exemple ou niveau d'une veine ou d'une artère d'un muscle affaibli. Cette technique d'administration, qui implique une administration locale qui produit des effets systémiques, généralement appelée administration loco-régionale, s'est avérée particulièrement adaptée pour le traitement des pathologies musculaires. En pratique, il peut s'agir d'une administration artérielle ou intraveineuse au niveau d'un membre du patient (jambe ou bras), réalisée sous pression grâce à la pose d'un garrot, comme décrit par exemple par Zheng et al. (Mol. Therapy, 2012, 20(2) : 456-461), Toromanoff et al. (Mol. Therapy, 2008, 16(7) : 1291-99) et Arruda et al. (Blood, 2010, 115(23) : 4678-88).

Outre l'administration loco-régionale, un mode d'administration privilégié selon l'invention est l'administration systémique. L'administration systémique permet d'atteindre le corps entier, et en particulier l'ensemble des muscles du patient, y compris le cœur et le diaphragme.

Selon un mode de réalisation privilégié, l'administration systémique est réalisée via l'injection de la composition dans un vaisseau sanguin, à savoir via une administration intravasculaire (intraartérielle ou intraveineuse). En particulier, la composition peut être administrée par injection intraveineuse, dans une veine périphérique. Alternativement, l'administration systémique peut être une injection intramusculaire.

De manière connue de l'homme du métier, l'administration systémique est réalisée dans les conditions classiques suivantes :
- Un débit d'écoulement compris entre 1 et 10 ml/min, avantageusement entre 1 et 5 ml/min, par exemple 3 ml/min ;
- Un volume d'injection total compris entre 1 et 10 ml, préférentiellement égal à 5 ml de la préparation vectorielle par kg du patient. Le volume injecté ne doit préférentiellement pas représenter plus de 10% du volume sanguin total, préférentiellement environ 6%.

Une administration unique de la composition selon l'invention peut s'avérer suffisante. Toutefois, plusieurs administrations dans différentes conditions peuvent être envisagées, notamment :
- Administration répétée du même vecteur par la même voie d'administration ;
- Administration du même vecteur au niveau de différents sites, en particulier au niveau de différents membres ;
- Administration de vecteurs recombinants différents pouvant varier au niveau de leur sérotype ou de la séquence codant l'AON délivré.

Selon un mode de réalisation, la présence du vecteur AAV selon l'invention, et les effets thérapeutiques bénéfiques associés, sont observés pour au moins 1 mois, ou 3 mois, ou 6 mois, ou 1 an, ou 2 ans, ou 5 ans ou 10 ans, ou même pendant toute la durée de vie du patient.

Comme déjà dit, le patient est avantageusement un être humain, en particulier un enfant, un adolescent ou un jeune adulte. Toutefois, l'outil thérapeutique selon l'invention peut être adapté et utile pour le traitement d'autres animaux, en particulier le porc et la souris.

De tels médicaments sont notamment destinés au traitement des maladies dystrophiques. Dans le cadre de l'invention, une maladie dystrophique désigne une maladie liée à un défaut dans le gène de la dystrophine. Plus précisément, il s'agit d'un défaut pour lequel le saut de l'exon 53 permet de rétablir le cadre de lecture et la production d'une dystrophine certes tronquée mais fonctionnelle. En rapport avec l'invention, une dystrophine tronquée mais fonctionnelle désigne une protéine de taille inférieure à la protéine native, en particulier ne comprenant pas la région codée par l'exon 53, mais qui est capable d'assurer au moins certaines des fonctions de la protéine native, et notamment d'atténuer au moins partiellement les symptômes associés à l'absence de la dystrophine native, tels que la dégénération des fibres, l'inflammation, la nécrose, le remplacement du muscle par du tissu de cicatrisation ou adipeux, la faiblesse musculaire, les insuffisances respiratoire ou cardiaque, ainsi que la mort prématurée.

Selon un mode de réalisation particulier, la maladie dystrophique est la Dystrophie Musculaire de Duchenne (DMD ou maladie de Duchenne).

Sont particulièrement concernées les formes de la DMD associés à des délétions de l'exon 52 (Δ52), des exons 50 à 52 (Δ50-52), des exons 49 à 52 (Δ49-52), des exons 48 à 52 (Δ48-52), des exons 47 à 52 (Δ47-52), des exons 46 à 52 (Δ46-52), des exons 45 à 52 (Δ45-52), des exons 43 à 52 (Δ43-52) et des exons 10 à 52 (Δ10-52) ou à une duplication de l'exon 53. L'intérêt de la présente invention est illustré plus avant en rapport avec les délétions Δ 52 et Δ45-52, mais aussi Δ48-52 et Δ50-52.

La nature des délétions du gène de la dystrophine présentes chez un patient est aisément déterminée par l'homme du métier, par exemple par séquençage ou PCR.

Au vu des effets remarquables observés sur la restauration de la dystrophine dans les fibres musculaires atteintes de DMD, l'invention concerne également l'utilisation d'un vecteur AAVr tel que décrit ci-dessus ou d'une composition le contenant pour la fabrication d'un médicament, avantageusement destiné au traitement des maladies dystrophiques, en particulier les DMD, notamment les formes listées ci-dessus.

En d'autres termes, la présente invention fournit une méthode de traitement des maladies dystrophiques, en particulier telles que définies ci-dessus, comprenant l'administration à un sujet d'un tel vecteur AAV ou d'une composition le contenant.

### EXEMPLES DE REALISATION

L'invention et les avantages qui en découlent ressortiront mieux des exemples de réalisation suivants, à l'appui des figures annexées. En particulier, la présente invention est illustrée (exemple 1) en rapport avec des vecteurs AAV 2/8 comprenant diverses séquences codant pour des AON d'intérêt au sens de l'invention et un snRNA modifié murin de type U7, testés sur des myoblastes présentant une délétion de l'exon 52 (Δ52) ou des exons 45-52 (Δ45-52) du gène humain de la dystrophine. L'invention est en outre illustrée (exemple 2) en rapport avec un vecteur AAV 2/8 comprenant une séquence codant un AON particulier (JR53), et un snRNA modifié murin de type U7, testé sur des fibroblastes de patients myoconvertis (fibromyoblastes) présentant une délétion de l'exon 52 (Δ52), ou des exons 45-52 (Δ45-52), 48-52 (Δ48-52), ou encore 50-52 (Δ50-52), du gène humain de la dystrophine. Ceux-ci n'ont toutefois aucune portée limitative, et leur enseignement peut être étendu à d'autres vecteurs AAV, d'autres snRNA modifiés et des sujets atteints de la maladie de Duchenne résultant d'autres mutations.

### LEGENDES DES FIGURES

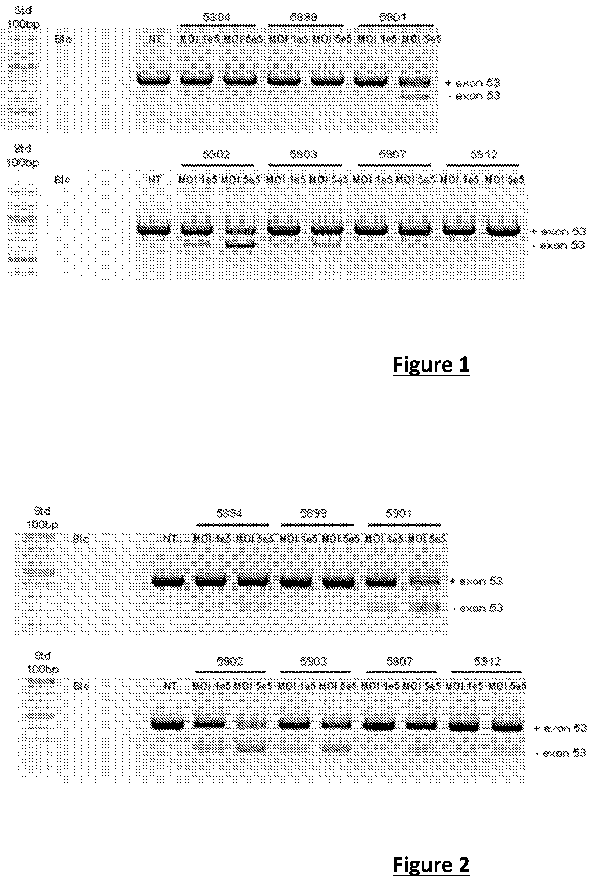

**Figure 1****:** Evaluation du saut de l'exon 53 du messager de la dystrophine humaine après transduction des myoblastes Δ45/52 avec les différents vecteurs rAAV8 testés en aveugle. L'analyse a été réalisée par RT-PCR nichée entre les exons 44 à 56. Blc : témoin eau de PCR ; NT : cellules non transduites.
**Figure 2****:** Evaluation du saut de l'exon 53 du messager de la dystrophine humaine après transduction des myoblastes Δ52 avec les différents vecteurs rAAV8 testés en aveugle. L'analyse a été réalisée par RT-PCR nichée entre les exons 51 à 54. Blc : témoin eau de PCR ; NT : cellules non transduites.

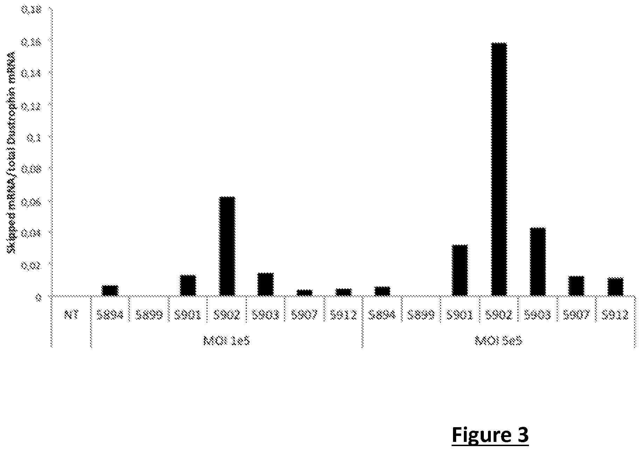

**Figure 3****:** Evaluation par RTqPCR du niveau d'expression du messager de la dystrophine humaine sans exon 53 (en fonction du niveau de messager de la dystrophine totale) après transduction des myoblastes Δ45/52 avec les différents vecteurs rAAV8 testés en aveugle. NT : cellules non transduites.

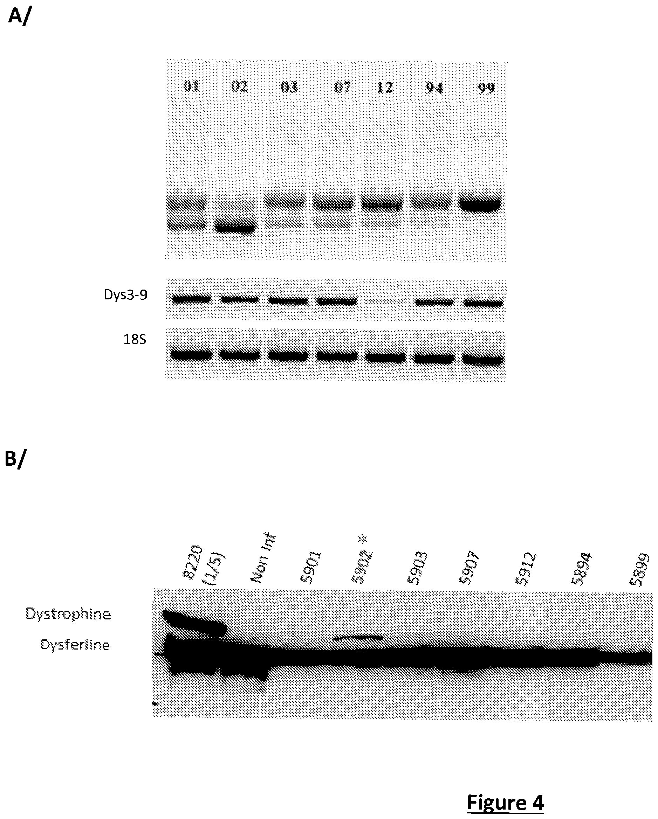

**Figure 4** : Saut de l'exon 53 du pré-ARNm de la dystrophine humaine sur myoblastes DMD Δ 45-52.
   **A**/ Les myoblastes Δ45-52 ont été transduits avec les vecteurs AAV2/8-U7-5901 (noté 01) ou 5902 (noté 02) ou 5903 (noté 03) ou 5907 (noté 07) ou 5912 (noté 12) ou 5894 (noté 94) ou 5899 (noté 99) (voir Matériel et Méthodes) à une MOI de 300.10E+3. Leur efficacité pour le saut de l'exon 53 a été évaluée par RT-PCR nichées. La quantité d'ARNm total de la dystrophine est évaluée par RT-PCR Dys3-9, et l'expression du gène de ménage 18S permet d'évaluer la présence d'ARNm.
   **B/** Les myoblastes Δ45-52 ont été transduits avec les vecteurs AAV2/8-U7-5901 ou 5902 ou 5903 ou 5907 ou 5912 ou 5894 ou 5899 (voir Matériel et Méthodes) à une MOI de 300.10E+3. Un western blot multiplex a été réalisé pour détecter les protéines dystrophine et dysferline sur les cellules de sujet sain (8220 ; extrait protéique dilué au 1/5) et les cellules DMD Δ45-52 non infectées (Non Inf). Cette figure est représentative de 4 expériences réalisées de façon indépendante.

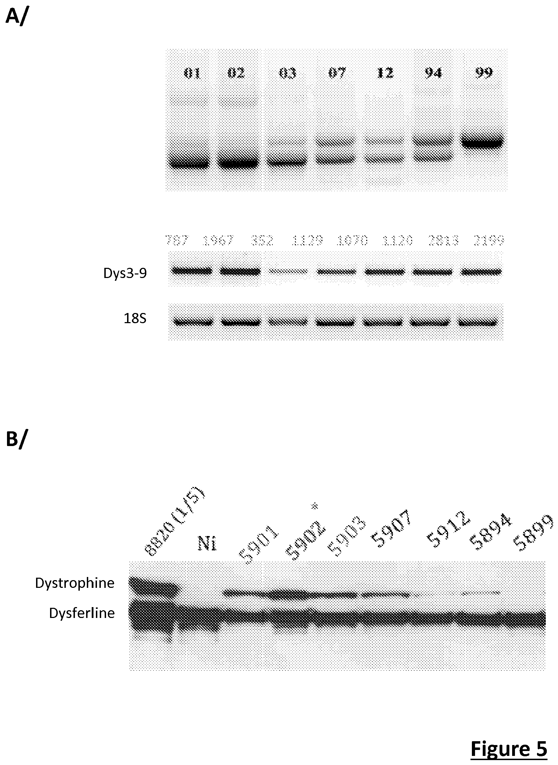

**Figure 5****:** Saut de l'exon 53 du pré-ARNm de la dystrophine humaine sur myoblastes DMD Δ 52.
   **A**/ Les myoblastes Δ52 ont été transduits avec les vecteurs AAV2/8-U7-5901 (noté 01) ou 5902 (noté 02) ou 5903 (noté 03) ou 5907 (noté 07) ou 5912 (noté 12) ou 5894 (noté 94) ou 5899 (noté 99) (voir Matériel et Méthodes) à une MOI de 300.10E+3. Leur efficacité pour le saut de l'exon53 a été évaluée par RT-PCR nichées. La quantité d'ARNm total de la dystrophine est évaluée par RT-PCR Dys3-9, et l'expression du gène de ménage 18S permet d'évaluer la présence d'ARNm.
   **B/** Les myoblastes Δ52 ont été transduits avec les vecteurs AAV2/8-U7-5901 ou 5902 ou 5903 ou 5907 ou 5912 ou 5894 ou 5899 (voir Matériel et Méthodes) à une MOI de 300.10E+3. Un western blot multiplex a été réalisé pour détecter les protéines dystrophine et dysferline sur les cellules de sujet sain (8220 ; extrait protéique dilué au 1/5) et les cellules DMD Δ52 non infectées (Ni). Cette figure est représentative de 4 expériences réalisées de façon indépendante.

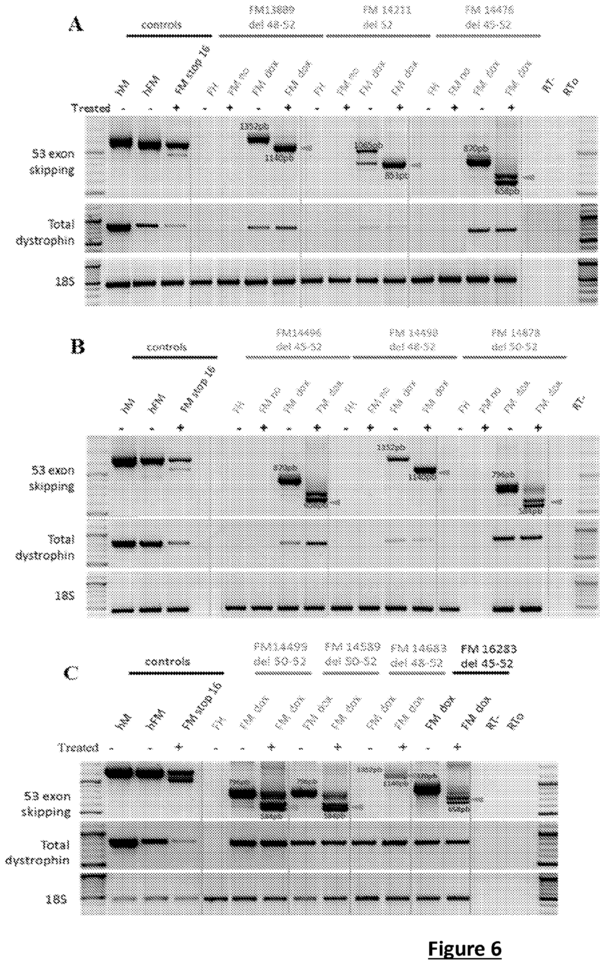

**Figure 6****:** Efficacité de JR53 pour le saut de l'exon 53 de la dystrophine dans des fibromyoblastes humains DMD transduits.
   A, de gauche à droite : marqueur de poids moléculaire ; myoblastes contrôles sains (hM) ; fibromyoblastes (hFM) ; fibromyoblastes DMD non éligibles (FMstop16) ; cellules de 3 patients DMD 5-13889 (FM13889 del49-52), 5-14211 (FM14211 del52), 5-14476 (FM14476 del45-52) avec pour chaque patient, 4 conditions : fibroblastes (FH), fibromyoblastes non induits par la doxycycline (FM no), fibromyoblastes induits par la doxycycline (FM dox-), fibromyoblastes induits par la doxycycline et traités par JR53 (FM dox+); contrôle négatif de la transcription inverse sans enzyme superscript (RT-), ou sans ARN (RTo) ; marqueur de poids moléculaire.
   B : identique à A avec les patients DMD 5-14496 (FM14496 del45-52), 5-14498 (FM14498 del48-52), 5-14878 (FM14878 del50-52) et sans le témoin RTo.
   C, de gauche à droite : marqueur de poids moléculaire ; myoblastes contrôles sains (hM) ; fibromyoblastes (hFM) ; fibromyoblastes DMD non éligibles (FMstop16) ; fibroblastes DMD contrôles (FH), fibromyoblastes induits par la doxycycline de 4 patients DMD 5-14499 (FM14499 del50-52), 5-14589 (FM14589 del50-52), 5-14683 (FM14683 del48-52), 5-16283 (FM16283 del45-52), avec pour chacun 2 conditions : non traités par JR53 (FM dox-), traités par JR53 (FM dox+) ; contrôle négatif de la transcription inverse sans enzyme superscript (RT-) ou sans ARN (RTo) ; marqueur de poids moléculaire.
      Les produits de RT-PCR ayant la taille attendue en l'absence d'exon 53 sont notés par une flèche.

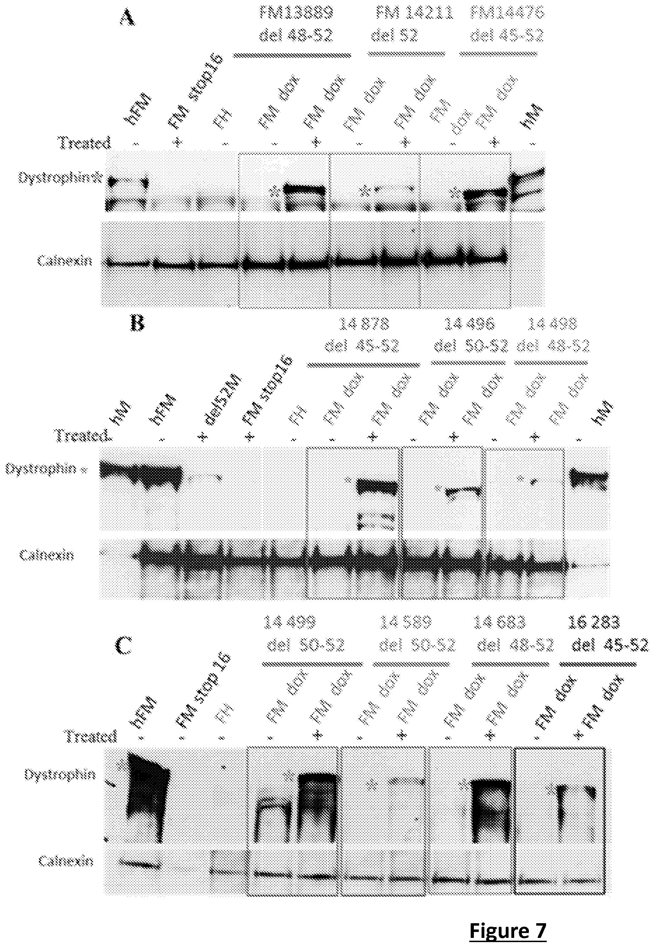

Figure 7: Expression de la protéine dystrophine dans les fibromyoblastes DMD humains transduits révélée par western blot.
   La détection de l'expression de la dystrophine (en haut) et de la calnexine (en bas) a été réalisée par western blot.
   A, de gauche à droite : fibromyoblastes contrôles sains (hFM) ; fibromyoblastes DMD non éligibles (FMstop16) ; fibromyoblastes DMD non induits par la doxycycline (FH); fibromyoblastes induits par la doxycycline de 3 patients DMD 5-13889 (FM13889 del48-52), 14211 (FM14211 del52), 5-14476 (FM14476 del45-52), chacun dans 2 conditions : non traités par JR53 (FM dox-) et traités par JR53 (FM dox+) ; myoblastes sains (hM).
   B : identique à A avec les patients DMD 5-14878 (FM14878 del45-52), 5-14496 (FM14496 del50-52), 5-14498 (FM14498 del48-52).
   C : identique à A avec les patients DMD 5-14499 (FM14499 del50-52), 5-14589 (FM14589 del50-52), 5-14683 (FM14683 del48-52), 5-16283 (FM16283 del45-52).
      L'efficacité de JR53 a été testée dans deux ou trois expériences pour chaque lot de fibromyoblastes DMD

### EXEMPLE 1 : EFFICACITE DE VECTEURS COMPRENANT DES SEQUENCES CODANT POUR DES OLIGONUCLEOTIDES ANTISENS (AON) DANS LE SAUT DE L'EXON 53 DE LA DYSTROPHINE SUR DES MYOBLASTES

L'efficacité des AAV8 recombinants décrits plus en détail ci-dessous a été testée en aveugle dans le cadre de deux études menées en parallèle.

La première étude (I) a eu pour but de comparer l'efficacité des différentes séquences antisens au niveau du saut de l'exon 53 (évaluée par RT-PCR sur l'ARNm de la dystrophine), en termes qualitatif et quantitatif.

La seconde étude (II) a eu pour but de confirmer l'efficacité des différentes séquences antisens au niveau du saut de l'exon 53 (évaluée par RT-PCR sur l'ARNm de la dystrophine) et au niveau de la protéine dystrophine produite.

### A. MATÉRIELS ET MÉTHODES

### 1/ Antisens, vecteurs et plasmides

Les séquences antisens identifiées dans le Tableau 1 ci-dessous ont été testées :

| **Région cible dans l'exon 53** | **Séquence de l'oligonucléotide antisens (AON)** | **Nom de l'AON** |
|---|---|---|
| (séquence sans homologie avec l'exon 53) | SEQ ID NO : 9 | 5899 (scramble) |
| + 33 à +59 | SEQ ID NO : 6 | 5894 (N1) |
| + 36 à +59 | SEQ ID NO : 5 | 5907 (N2) |
| + 33 à +65 | SEQ ID NO : 4 | 5901 (N3) |
| + 36 à +65 | SEQ ID NO : 7 | 5903 (Di I) |
| + 30 à +69 | SEQ ID NO : 3 | 5902 (JR53) |
| + 45 à +62 et +128 à +145 | SEQ ID NO : 8 | 5912 (Dtex53) |

Elles ont été clonées par mutagénèse dirigée dans le gène U7snRNA murin optimisé et contenant son propre promoteur. La construction correspondante en rapport avec l'AON 5902 (JR53) est illustrée à la séquence SEQ ID NO : 2.

Les constructions correspondantes ont été insérées dans un plasmide d'expression, par exemple le plasmide pFBD.

Comme décrit par Ayuso et al. (Hum. Gen. Ther., 2014, Nov 25(11): 977-87), les plasmides utilisés pour la production des vecteurs AAV2/8 sont :
- pDP10 portant les gènes rep-2 et cap-8 et les gènes auxiliaires de l'adénovirus E2b, E4, VARNA ; et
- le plasmide vecteur portant les ITR-2 correspondants et le transgène (constructions décrites ci-dessus).

### 2/ Production de vecteurs AAV2/8

Les vecteurs AAV8 ont été produits à partir des cellules HEK293 (ayant intégré en stable dans leur génome cellulaire les gènes E1A et E1B) cultivées en système adhérent en Cell Stack 5 plateaux. Les cellules, cultivées dans du milieu DMEM contenant 10 % de sérum de veau fœtal (volume/volume) ainsi que de la pénicilline et streptomycine (1% volume/ volume pour chaque antibiotique), ont été transfectées par la technique de précipitation au phosphate de Calcium de 2 plasmides (plasmide auxiliaire: pDP10 portant les gènes rep-2 et cap-8 et les gènes auxiliaires de l'adénovirus E2b, E4, VARNA et le plasmide vecteur portant les ITR-2 correspondant et le transgène).

Le milieu est changé après la transfection par du milieu DMEM sans sérum. Les particules AAVr sont récoltées après 72 H dans le surnageant et précipitées au PolyEtheneGlycol 40% plus benzonase Les particules sont ensuite purifiées sur 2 gradients successifs de CsCl. Les contaminations en CsCl sont éliminées par dialyse contre du tampon dPBS 1X. Les particules sont ensuite congelées à -80°C dans des tubes « low binding » (Ayuso et al., Hum. Gen. Ther., 2014, Nov 25(11) : 977-87).

### 3/ Titration des vecteurs

Les productions de vecteurs sont traitées à la DNAse pour éliminer l'ADN libre contaminant puis on procède à une extraction des acides nucléiques viraux. Après extraction, les acides nucléiques sont dilués et le nombre de copies de génomes viraux AAV (amplicon AAV/ITR) est déterminé par qPCR avec une gamme plasmide linéarisée et standardisée.

Pour la qPCR, les amorces et sonde suivantes sont utilisées :
AAV18mers.R : GTAGATAAGTAGCATGGC (SEQ ID NO : 11)
AAV22mers.F : CTCCATCACTAGGGGTTCCTTG (SEQ ID NO : 12)
AAV_M G B.P: TAGTTAATGATTAACCC (SEQ ID NO : 13).

### 4/ Lignées de myoblastes immortalisées

Deux lignées de patients Duchenne ont été utilisées, l'une portant une délétion dans le gène de la dystrophine des exons 45 à 52 (Δ45-52) et l'autre de l'exon 52 (Δ52). Une troisième lignée de myoblastes de sujet sain a servi de contrôle (8220).

### I/ Protocole mis en œuvre dans le cadre de l'étude I :

### 5/ Transduction des myoblastes immortalisés par AAV2/8

Les cellules sont ensemencées à 190 000 cellules par puits de plaque 6 puits, puis cultivées dans le milieu de prolifération (skeletal muscle cell basal medium + skeletal muscle cell growth medium kit, Promocell Ref : C23160). Après 24 heures environ, les cellules sont transduites aux MOI de 1^{e}5 et 5^{e}5 vg/cellule avec les différents vecteurs AAV2/8 recombinants U7snRNA modifiés en aveugle et un témoin AAV2/8 permettant l'expression de la Green fluoresent protein (GFP) afin de vérifier l'efficacité des transductions.

Après 3 jours de culture, les cellules sont récoltées et les culots cellulaires sont congelés à sec à -80°C. Les culots sont ensuite utilisés pour les purifications d'ADN et d'ARN.

### 6/ Evaluation du nombre de génomes viraux par cellule

L'extraction des génomes AAV est réalisée en utilisant le kit Gentra Puregene kit (Qiagen). Le nombre de copies d'ADN de vecteur est déterminé en utilisant la procédure TaqMan Real time PCR. Les amorces et sonde sont élaborées pour amplifier (i) les Inverted Terminal Repeats (ITRs) (voir le point 3) et (ii) le gène endogène de l'albumine. Pour chaque échantillon, les valeurs de Ct sont comparées à celles obtenues avec différentes dilutions d'un plasmide standard contenant à la fois les ITRs et le gène albumine. Les résultats finaux sont exprimés en génomes viraux par génome diploïde (vg/dg pour « viral genome/ diploid genome »).
Alb.F : GCTGTCATCTCTTGTGGGCTGT (SEQ ID NO : 14)
Alb.R : ACTCATGGGAGCTGCTGGTTC (SEQ ID NO : 15)
AlbVIC.P : CCTGTCATGCCCACACAAATCTCTCC (SEQ ID NO : 16)

### 7/ Evaluation du saut d'exon par RT-PCR nichée

L'extraction d'ARN à partir du culot sec de cellules est réalisée avec 1mL de Trizol Reagent selon le mode opératoire du fournisseur (Life Technologies, Ref 15596-026). Les ARN sont repris dans 30µl d'eau stérile puis sont traités par deux étapes successives de DNase afin d'éliminer les contaminations habituelles des ARN par les ADN viraux (Ambion, Ref AM1907). 500 ng d'ARN sont utilisés pour la réaction de transcription reverse (kit Life Technologies, Ref 28025-013). Lors de cette étape des contrôles négatifs de reverse transcription sont réalisés sur chaque échantillons afin de vérifier l'absence de contamination par des ADN viraux.

Pour la détection du saut d'exon, une première PCR (PCR1) avec l'enzyme Gotaq G2 (Promega, Ref : M7805) est effectuée à partir de 1µl d'ADN complémentaires (ADNc) dans un volume final de 50 µl avec les couples d'amorces appropriés à chaque type cellulaire (voir la liste des amorces ci-dessous) pour 25 cycles [95°C 30secondes - 50°C ou 58°C 1min- 72°C 2min], puis 1µl de la PCR1, dans un volume de 50 µl total, sont ré-amplifiés (PCR2) pour 30 cycles [95°C 30secondes - 50°C ou 58°C 1min- 72°C 2min] avec un deuxième couple d'amorces internes au produit de PCR1. L'analyse des produits de PCR nichée est réalisée par électrophorèse sur gel d'agarose 1,5% dans du tampon Tris-acétate-EDTA. Les bandes correspondant au saut d'exon sont découpées, purifiées sur colonne, à l'aide du Kit Nucleospin Gel and PCR Clean-up (MACHEREY-NAGEL, Ref: 740609.250) et séquencées.

Les couples d'amorces utilisés pour le saut d'exon 53 sont :

### Pour la lignée DMD Δ52 :

PCR1 : ex51 hDMD Fext et ex54 hDMD Rext à 50°C
PCR2 : ex51 hDMD Fint et ex54 hDMD Rint à 50°C

### Pour la lignée Δ45-52:

PCR1 Dys43F/Dys57R à 58°C
PCR2 Dys44F/Dys56R à 58°C

### Séquences des amorces pour le saut d'exon 53 :

ex51 hDMD Fext: GTTACTCTGGTGACACAACC (SEQ ID NO : 17)
ex54 hDMD Rext : ATGTGGACTTTTCTGGTATC (SEQ ID NO : 18)
ex51 hDMD Fint : ACTAGAAATGCCATCTTCCT (SEQ ID NO : 19)
ex54 hDMD Rint : CAAGTCATTTGCCACATCTA (SEQ ID NO : 20)
Dys43-F : CCTGTGGAAAGGGTGAAGC (SEQ ID NO : 21)
Dys44-F : CGATTTGACAGATCTGTTGAG (SEQ ID NO : 22)
Dys56-R : TGAGAGACTTTTTCCGAAGT (SEQ ID NO : 23)
Dys57-R: AAGTTCCTGCAGAGAAAGGT (SEQ ID NO : 24)

Les tailles attendues pour la PCR2 sont indiquées dans le Tableau 2 ci-dessous.

| **Cellules** | Amorces | Taille de l'amplicon contenant l'exon 53 | Taille de l'amplicon ne contenant pas l'exon |
|---|---|---|---|
| Δ52 | ex51 hDMD Fint ex54 hDMD Rint | 438 bp | 226 pb |
| Δ45-52 | Dys44-F Dys56-R | 870 pb | 658 pb |

### 8/ Quantification du saut d'exon 53 par PCR quantitative dans la lignée Δ45-52

Deux RTqPCR sont réalisées afin de quantifier le saut de l'exon 53. La première est spécifique du transcrit ne contenant plus l'exon 53 de la dystrophine (RTqPCR de la jonction des exons 44 et 54). La deuxième est utilisée pour normaliser en amplifiant tous les transcrits de la dystrophine (RTqPCR de la jonction des exons 4 et 5). Les ADNc issus de la réaction de transcription reverse sont dilués puis amplifiés à l'aide kit Premix Ex Taq utilisant la technologie Taqman selon les recommandations du fournisseur (Takara, Ref RR390w) avec 0.3µmol/l de chaque amorce, et 0.25µmol/l de la sonde Taqman (voir la liste des amorces et des sondes ci-dessous) pour 40 cycles [95°C 15secondes - 57°C 1min]. Pour valider l'efficacité des RTqPCR et quantifier les transcrits, des gammes de dilutions d'un plasmide de référence contenant les séquences d'intérêt ont été déposées en parallèle des échantillons sur la plaque.

Pour la RTqPCR dystrophine sans l'exon 53, les amorces et sonde suivantes sont utilisées :
hDys-44/54-F : CCTGAGAATTGGGAACATGCTAA (SEQ ID NO : 25)
hDys-44/54-R : GCCACTGGCGGAGGTCTT (SEQ ID NO : 26)
hDys-44/54-P : GGTATCTTAAGCAGTTGGC (SEQ ID NO : 27) = sonde Taqman chevauchant la jonction des exons 44 et 54
Pour la RTqPCR dystrophine totale, les amorces et sonde suivantes sont utilisées : hDys-4/5-F : CATGCCCTGAACAATGTCAACAAG (SEQ ID NO : 28)
hDys-4/5-R : TCCATCTACGATGTCAGTACTTCCA (SEQ ID NO : 29)
hDys-4/5-P : TTGCAGAACAATAATGTTGATTTA (SEQ ID NO : 30) = sonde Taqman chevauchant la jonction des exons 4 et 5

### II/ Protocole mis en œuvre dans le cadre de l'étude II :

### 5/ Transduction des myoblastes immortalisés par AAV2/8

Les cellules sont ensemencées à 100 000 cellules par puits de 35 mm, puis cultivées dans le milieu de prolifération susmentionné jusqu'à 80% de confluence. Après 24 heures environ, les cellules sont infectées avec les différents vecteurs AAV2/8 recombinants à la MOI de 250 000 vg/cellule dans du milieu de différentiation (IMDM sans sérum avec gentamycine), à 37°C.

Après 4h à 6h, 250µL de IMDM additionné de gentamycine est ajouté. Après 9 jours de culture en milieu de différenciation, un échantillon du milieu de culture de 1/10 du volume du puit de 35 mm est prélevé. Il sera ensuite utilisé pour la purification d'ARN pour les analyses par RT-PCR. Parallèlement, les cellules sont grattées, rincées et centrifugées 1 minute à 11 000g ou 5 minutes à 1500 rpm. Les culots sont congelés à sec à -80°C. Les culots sont ensuite utilisés pour la purification d'ADN et la quantification de génome viral.

### 6/ Evaluation du nombre de génomes viraux par cellule

Voir point I-6 ci-dessus.

### 7/ Evaluation du saut d'exon par RT-PCR nichée

Afin de procéder à la RT-PCR nichée, l'ARN est extrait sur colonne à l'aide du kit Nucleospin RNAII et élué dans 30µl d'eau (DNAse/RNAse free). 500 ng d'ARN sont utilisés pour la réaction de transcription reverse (kit Superscript II Life Technologies, Ref 18064-014).

Pour la détection du saut d'exon, une 1ère PCR est effectuée sur 2µl de cDNA dans un volume de 20 µl total avec les couples d'amorces appropriés à chaque type cellulaire (voir la liste ci-dessous) pour 30 cycles [95°C 30" - 56°C 1'- 72°C 2'], puis 3µl de la PCR1, dans un volume de 30 µl total, sont amplifiés pour 25 cycles avec un deuxième couple d'amorces internes au produit de PCR1. Concernant l'analyse des ARNs 18S et PO, 1 seule PCR est effectuée. L'analyse des produits de PCR nichée est réalisée par électrophorèse sur gel d'agarose 1,8% dans du tampon Tris-acétate-EDTA. Les bandes correspondant au saut d'exon sont découpées, purifiées sur colonne, à l'aide du Kit Nucleospin Gel and PCR Clean-up (MACHEREY-NAGEL, Ref: 740609.250) et séquencées (par GATC).

Les couples d'amorces utilisés pour le saut d'exon 53 sont :

### Pour les myoblastes contrôles wt (8220) et DMD Δ52 :

PCR1 : Dys49F/Dys57R
PCR2 : Dys50F/Dys56R (dystrophine humaine)

### Pour les myoblastes Δ45-52 :

PCR1 Dys43F/Dys57R
PCR2 Dys44F/Dys56R (dystrophine humaine)

### Séquences des amorces 5'->3'pour le saut d'exon 53 :

Dys43-F : CCTGTGGAAAGGGTGAAGC (SEQ ID NO : 21)
Dys44-F : CGATTTGACAGATCTGTTGAG (SEQ ID NO : 22)
Dys49-F : CAACCGGATGTGGAAGAGAT (SEQ ID NO : 31)
Dys50-F : CTCTGAGTGGAAGGCGGTAA (SEQ ID NO : 32)
Dys56-R : TGAGAGACTTTTTCCGAA-GT (SEQ ID NO : 23)
Dys57-R: AAGTTCCTGCAGAGAAAG-GT (SEQ ID NO : 24)

### Séquences des autres amorces pour amplification 18S, PO et dystrophine humaine totale:

PO -F: GGCGAGCTGGAAGTGCAACT (SEQ ID NO : 33)
PO-R: CCATCAGCACCACAGCCTTC (SEQ ID NO : 34)
18S-F: TCAAGAACGAAAGTCGGAGGTTCG (SEQ ID NO : 35)
18S-R : TTATGCTCAATCTCGGGTGGCTG (SEQ ID NO : 36)
Dys3-F : GAGAACCTCTTCAGTGACCTAC (SEQ ID NO : 37)
Dys9-R : GAGGTGGTGACATAAGCAGC (SEQ ID NO : 38)

Les amorces utilisées pour chaque type cellulaire et les tailles attendues sont indiqués dans le Tableau 3 ci-dessous.

| **Cellules** | Amorces | Taille bande parentale (amplicon avec exon 53) | Taille bande attendue (amplicon sans exon 53) |
|---|---|---|---|
| **8220 Contrôle** | Dys43-F-Dys57-R Dys44-F-Dys56-R | 2010 pb | 1888 pb |
| **Δ45-52** | Dys43-F-Dys57-R Dys44-F-Dys56-R | 870 pb | 658 pb |
| **Δ52** | Dys49-F-Dys57-R Dys50-F-Dys56-R | 1065 pb | 853 |

### 8/ Evaluation de la restauration de l'expression de la dystrophine par western blot multiplex

Les protéines sont extraites à partir des cellules transduites et différenciées pendant 9 jours (voir paragraphe 5) dans un tampon contenant 125 mM de sucrose, 5 mM de Tris-HCI pH 6.4, 6% de Tampon de migration XT-Tricine (Bio-Rad), 10% de SDS, 10% de Glycérol, 5% de 13- mercaptoéthanol et d'antiprotéases. Les échantillons sont dénaturés 5 minutes à 95°C puis pré-traités avec le Kit Compat-AbleTM Protein Assay preparation Reagent Set (Thermo Scientific Pierce). La concentration des protéines est déterminée par le BCA Protein Assay Kit (Thermo Scientific Pierce). 75 µg de protéines sont déposées sur un gel Criterion XT Tris-Acetate 3-8% pré-coulé (Bio-Rad). La dystrophine est détectée par hybridation de la membrane avec l'anticorps primaire monoclonal NCL-DYS1 (Novocastra) dilué au 1:50 suivie par une incubation avec l'anticorps secondaire sheep anti-mouse (HRP), dilué au 1:15000. Les protéines sont révélées par le kit SuperSignal West Pico Chemiluminescent Substrate (Thermo Scientific Pierce).

### B. RÉSULTATS ET DISCUSSION

Une étude en double-aveugle a été menée avec les séquences antisens candidates, insérées dans le gène U7snRNA murin optimisé et contenant son propre promoteur, portés par les AAV2/8, sur deux lignées de myoblastes humains immortalisés, Δ45-52 et Δ52 respectivement.

Les vecteurs testés et leur titre sont indiqués dans le Tableau 4 ci-dessous :

| **Vecteur** | **Titre (vg/ml) (qPCR ITR-G)** |
|---|---|
| rAA V2/8-U7-#5899 | 1,20⁺¹² |
| rAAV2/-U7-#5894 | 7,54⁺¹¹ |
| rAAV2/8-U7-#5907 | 2,70⁺¹² |
| rAA V2/8-U7-#5901 | 2,00⁺¹² |
| rAAV2/8-U7 #5903 | 1,85⁺¹² |
| rAA V2/8-U7-#5902 | 9,60⁺¹¹ |
| rAAV2/8-U7 #5912 | 3,10⁺¹² |
| rAAV2/8 GFP | 1,14⁺¹² |

### I/ Résultats de l'étude I :

Ces vecteurs ont été testés à une MOI de 1^{e}5 et 5^{e}5 vg/cellule en condition de prolifération en aveugle par deux manipulateurs. L'efficacité des transductions est estimée par cytométrie en flux et se trouve entre 68% à 96% de cellules transduites.

Comme montré dans le Tableau 5 ci-dessous, la quantification par qPCR des génomes viraux par cellule met en évidence l'homogénéité de transduction des différents vecteurs :

| **Lignée** | **Vecteur** | **MOI** | **VG/DG** |
|---|---|---|---|
| **Δ45/52** | NT | / | 0,003 |
| | GFP | 1E+05 | 844 |
| | | 5E+05 | 3 416 |
| | 5894 | 1E+05 | 618 |
| | | 5E+05 | 3 266 |
| | 5899 | 1E+05 | 716 |
| | | 5E+05 | 3 081 |
| | 5901 | 1E+05 | 1 367 |
| | | 5E+05 | 5 832 |
| | 5902 | 1E+05 | 979 |
| | | 5E+05 | 16 103 |
| | 5903 | 1E+05 | 1 304 |
| | | 5E+05 | 5 592 |
| | 5907 | 1E+05 | 321 |
| | | 5E+05 | 2 978 |
| | 5912 | 1E+05 | 554 |
| | | 5E+05 | 2 663 |
| **Δ52** | NT | / | 0,006 |
| | GFP | 1E+05 | 2 925 |
| | | 5E+05 | 16 057 |
| | 5894 | 1E+05 | 3 373 |
| | | 5E+05 | 17 311 |
| | 5899 | 1E+05 | 2 474 |
| | | 5E+05 | 16 950 |
| | 5901 | 1E+05 | 2 697 |
| | | 5E+05 | 24 826 |
| | 5902 | 1E+05 | 4 118 |
| | | 5E+05 | 37 806 |
| | 5903 | 1E+05 | 2 628 |
| | | 5E+05 | 24 201 |
| | 5907 | 1E+05 | 1 557 |
| | | 5E+05 | 10 808 |
| | 5912 | 1E+05 | 1 915 |
| | | 5E+05 | 10 264 |

Les figures 1 à 3 présentent les résultats de l'évaluation de l'efficacité des constructions après transduction des myoblastes DMD Δ45-52 et Δ52 respectivement : Les figures 1 et 2 permettent d'apprécier le saut de l'exon 53 par RT-PCR nichées sur les deux lignées et la figure 3 présente la quantification de ce saut par RTqPCR sur la lignée Δ45-52.

### II/ Résultats de l'étude II :

Ces vecteurs ont été testés à une MOI de 300.E+3. Les cellules transduites ont été mises en différenciation pendant 9 jours afin d'obtenir les conditions de différenciation permettant la mise en évidence d'une restauration de la protéine dystrophine dans les cellules corrigées (voir Matériel et Méthodes).

Les figures 4 et 5 présentent les résultats de l'évaluation de l'efficacité des constructions après transduction des myoblastes DMD Δ45-52 et Δ52 respectivement.

Les figures 4A et 5A permettent d'apprécier le saut de l'exon 53 par RT-PCR nichées et les figures 4B et 5B présentent les résultats de l'étude de l'expression de la protéine dystrophine par western blot.

### CONCLUSIONS :

Il ressort de ces deux études indépendantes et tout à fait cohérentes, que les résultats de l'efficacité du saut d'exon sont identiques pour les deux lignées DMD, à savoir une efficacité dans l'ordre suivant : 5902>5901=5903. De même, sur les deux lignées de myoblastes, la construction qui porte l'AON 5902 est la plus efficace pour restaurer l'expression de la dystrophine.

Les séquences antisens clonées dans U7 et vectorisées en AAV2/8, en particulier les séquences appelées JR53 (SEQ ID NO : 3) et N3 (SEQ ID NO : 4), sont toutes plus efficaces que la séquence Dtex53 selon l'art antérieur (5912).

### EXEMPLE 2 : EFFICACITE DE VECTEURS COMPRENANT LA SEQUENCE JR53 (SEQ ID NO : 3) DANS LE SAUT DE L'EXON 53 DE LA DYSTROPHINE, SUR DES FIBROMYOBLASTES HUMAINS

### A. MATÉRIELS ET MÉTHODES

### 1/ Culture de cellules

Les fibroblastes de patients DMD ont été cultivés dans un milieu de culture IMDM (pour « Iscove's Modified Dubelco Médium » ; Life Technologies) additionné de 10% de sérum de veau fœtal (SVF) et de 10 µg/ml de gentamycine. A confluence, les cellules sont dissociées dans une solution de trypsine/EDTA et amplifiées. Pour chaque patient, des échantillons ont été préparés et stockés au froid (cryoconservation).

### 2/ Myoconversion: conversion des fibroblastes DMD humains en fibromyoblastes

### 2.1 Vecteur utilisé pour la myoconversion

La technique de myoconversion et les outils permettant sa mise en œuvre, notamment les vecteurs lentiviraux adaptés, sont connus de l'homme du métier et par exemple décrits dans Cooper et al. (Neuromuscular Disorders 17 (2007), 276-284) et Chaouch et al. (Human Gene Therapy 20 (2009), 784-790).

Le vecteur utilisé pour la myoconversion est le vecteur lentiviral GU007HT _psin_pTRE_MyoD_hPGK_rtTA2M2, produit par Généthon (lot n °13RO281 GU 007H), également appelé par la suite LV/MyoD ou LV/MyoDi.

### 2.2 Méthode de myoconversion

Les fibroblastes humains (FH) ont été dissociés avec une solution de trypsine/EDTA, centrifugés pendant 5 minutes à 300 g, remis en suspension dans du milieu DMEM (« High glucose Dulbecco's Médium ») et comptés. 25 000 cellules ont été transférées dans un tube Eppendorf avec 5 µL de LV/MyoDi dans du DMEM sans antibiotique, dans un volume total de 100 µl, puis incubées pendant 30 minutes à 37 °C (avec 2 à 3 agitations). Ensuite, le mélange (cellules + vecteur) contenu dans chaque tube a été ensemencé dans un puits de 22 mm (P12) contenant 900 µl de DMEM, 10% SVF, 100 µl/ml de Pénicilline, 100 µg/ml de Streptomycine, et placé dans un incubateur à 37 °C, 5% de CO₂ pendant 3 jours. A partir du troisième jour, le milieu est remplacé intégralement pour éliminer les particules virales. Les cellules transduites par LV/MyoDi ont ensuite été amplifiées et congelées à -80 °C avant d'être transférées dans de l'azote liquide.

### 2.3 Validation de l'efficacité de la myoconversion par marquage immunocellulaire de MyoD1

Les fibromyoblastes (FM) ont été inoculés sur une lamelle de 22 mm sur matrigel à 10000 cellules/puits. La myoconversion est induite après 24h par l'addition de 10 µg/ml de doxycycline pendant 4 à 6 jours dans un milieu de prolifération. Les fibromyoblastes ont été fixés avec 2% de para-formaldéhyde (PFA) pendant 15 minutes à température ambiante (et, si nécessaire, maintenus à 4 °C). Les cellules ont été perméabilisées pendant 5 minutes avec du méthanol à -20 °C, bloquées pendant 1 heure dans du PBS comprenant 2% de BSA (« Bovine Serum Albumine »). Des marquages immunologiques ont été réalisés en utilisant l'anticorps monoclonal de souris MyoD1 clone 5.8A (code DAKO M3512) dilué au 1/100, incubé pendant une nuit à 4 °C. Ensuite, un anticorps secondaire Alexa fluoro 488-IgG anti-souris de chèvre (1/500) a été incubé pendant 1 heure à température ambiante. Les noyaux ont été marqués avec du DAPI pendant 5 minutes. Les lamelles ont été montées sur une lame Fluoromount G et observées sous un microscope à fluorescence (DM2500 Leica confocal imaging et application ImageJ).

L'efficacité de la myoconversion a été exprimée en calculant le pourcentage de noyaux positifs pour MyoD1 par rapport aux noyaux positifs au DAPI (100 cellules comptées par condition).

### 3/ Produit clinique

Le vecteur utilisé pour les essais cliniques, comprenant la séquence JR53 (SEQ ID NO : 3), est le vecteur G7U007 bacculoAAV2 / 8-U7-JR53 produit par Généthon (lot n ° 14PO353: JR53), comme décrit dans l'exemple 1.

### 4/ Transduction des fibromyoblastes DMD humains par JR53

Des fibroblastes myoconvertis (fibromyoblastes) ont été infectés par le vecteur comprenant la séquence JR53 (MOI 300 000 génomes viraux par cellule) dans du milieu IMDM sans sérum à 37 °C. Cinq heures plus tard, les fibromyoblastes ont été différenciés dans du milieu IMDM, additionné de 1% de sérum de cheval, 10 µg/ml de gentamycine et 10 µg/ml de doxycycline pendant 13 à 14 jours. Les cellules différenciées ont été grattées dans du PBS, à température ambiante, collectées dans un premier tube (destiné au transfert de type Western) et maintenues sur la glace. 1/10 de la quantité récoltée a été transférée pour l'extraction d'ARN. Après centrifugation de 1 minute à 11 000 g, les culots cellulaires ont été congelés et stockés à -80 °C.

### 5/ Test du saut d'exon sur les pré-ARNm de la dystrophine dans des fibromyoblastes DMD humains transduits

L'ARN total a été extrait des cellules à l'aide du kit Nucleospin RNAII (Macherey Nagel) selon les instructions du fabricant et élué dans 30 µl d'eau. Des parties aliquotes de 500 ng d'ARN total ont été utilisées pour une analyse RT-PCR nichée. La réaction de transcription inverse a été effectuée avec un analyseur d'amorce aléatoire à 42 °C pendant 50 min à l'aide du kit Superscript II (Life Technologies), puis par PCR en utilisant le kit PCR master mix (Promega).

Pour l'évaluation du saut d'exon du pré-ARNm de la DMD, une RT-PCR nichée a été réalisée (PCR1): 2µl d'ADNc dans un volume total de 20µl avec des amorces entourant la mutation et l'exon 53 (voir Tableau 6), pendant 30 cycles [95 °C (30 sec) - 56 °C (1 min) - 72 °C (2 min)]. Ensuite, 3 µl de la PCR1 dans un volume total de 30 µl ont été amplifiés pendant 25 cycles avec une 2ème paire d'amorces (voir Tableau 6).

Pour l'évaluation de l'ARNm total de la dystrophine et de l'ARNm ribosomal 18S (contrôle endogène de l'ajout d'ADNc), une PCR unique de 34 cycles [95 °C (30 sec) - 57 °C (1 min) - 72 °C (1 min)] a été réalisée avec la paire d'amorces décrite dans le Tableau 6.

Les produits de PCR ont été analysés par électrophorèse sur gel de 1,8% d'agarose dans du tampon Tris-acétate-EDTA. Les tailles attendues des produits de RT-PCR sont présentées dans le Tableau 7 ci-dessous. Le nombre de pixels obtenus pour chaque bande a été traité avec le logiciel ImageJ. Les bandes correspondant à la taille de l'amplicon présentant un saut d'exon (bande inférieure) ont été découpées, purifiées sur colonne en utilisant le Kit Nucleospin Gel et PCR Clean-up (Macherey Nagel), et séquencées (par GATC).

**Tableau 6 : Liste des amorces utilisées dans les expériences de PCR**

| **Saut d'exon** | **PCR1** | **PCR2** |
|---|---|---|
| Myoblastes sains et del52 | Dys49F/Dys57R | Dys50F/Dys56R |
| | Dys49-F (SEQ ID NO : 31) : | Dys50-F (SEQ ID NO : 32) : |
| | CAACCGGATGTGGAAGAGAT | CTCTGAGTGGAAGGCGGTAA |
| | Dys57-R (SEQ ID NO : 24) : | Dys56-R (SEQ ID NO : 23) : |
| | AAGTTCCTGCAGAGAAAG-GT | TGAGAGACTTTTTCCGAA-GT |
| Fibroblastes et fibromyoblastes del50-52 | Dys49F/Dys57R | Dys49F/Dys56R |
| | Dys49-F (SEQ ID NO : 31) : | Dys49-F (SEQ ID NO : 31) : |
| | CAACCGGATGTGGAAGAGAT | CAACCGGATGTGGAAGAGAT |
| | Dys57-R (SEQ ID NO : 24) : | Dys56-R (SEQ ID NO : 23) : |
| | AAGTTCCTGCAGAGAAAG-GT | TGAGAGACTTTTTCCAA-GT |
| Fibroblastes et fibromyoblastes del 48-52 ou del45-55 | Dys 43F/Dys57R | Dys44F/Dys56R |
| | Dys43-F (SEQ ID NO : 21) : | Dys44-F (SEQ ID NO : 22) : |
| | CCTGTGGAAAGGGTGAAGC | CGATTTGACAGATCTTGTTGAG |
| | Dys57-R (SEQ ID NO : 24) : | Dys56-R (SEQ ID NO : 23) : |
| | AAGTTCCTGCAGAGAAAG-GT | TGAGAGACTTTTTCCGAA-GT |
| ARNm dystrophine totale | **PCR** | |
| | Dys3-F/Dys9-R | |
| | Dys3-F (SEQ ID NO : 37) : | |
| | GAGAACCTCTTCAGTGACCTAC | |
| | Dys9-R (SEQ ID NO : 38) : | |
| | GAGGTGGTGACATAAGCAGC | |
| ARNm ribosomal 18s | **PCR** | |
| | 18S-F/18S-R | |
| | 18S-F (SEQ ID NO : 35) : | |
| | TCAAGAACGAAAGTCGGAGGTTCG | |
| | 18S-R (SEQ ID NO : 36) : | |
| | TTATGCTCAATCTCGGGTGGCTG | |

**Tableau 7 : Taille prévue des produits de RT-PCR**

| | Saut de l'exon 53 exon | | | Dystrophine Totale | 18S |
|---|---|---|---|---|---|
| Cellules | Amorces | Parental Taille de l'amplicon (bp) | del53 Taille de l'amplicon (bp) | Taille (pb) | Taille (pb) |
| Saines et contrôles | F49-R57 F50-R56 | 1183 | 971 | 818 | 459 |
| del 52 | F49-R57 F50-R56 | 1065 | 853 | 818 | 459 |
| del 50-52 | F49-R57 F49-R56 | 796 | 584 | 818 | 459 |
| del48-52 | F43-F57 F44-R56 | 1352 | 1140 | 818 | 459 |
| del 45-52 | F43-F57 F44-R56 | 870 | 658 | 818 | 459 |

### 6/ Test de l'expression de la protéine dystrophine par western blot dans les fibromyoblastes humains DMD transduits

Les protéines cellulaires ont été extraites dans du tampon de lyse (saccharose 125 mM, Tris-HCl 5 mm pH 6,4, tampon de migration de XT-tricine à 6% (Bio-Rad), SDS à 10%, glycerol à 10%, β-mercaptoéthanol à 5%, inhibiteurs de protéases). Les échantillons ont été dénaturés 5 minutes à 95 °C.

La concentration en protéines a été déterminée à l'aide du kit BCA Protein Assay Kit (Thermo Scientific Pierce) sur 2 µl d'échantillon de protéine prétraité par le réactif de dosage de protéine du kit Compat-Able ™ (Thermo Scientific Pierce). 75 à 100 µg de protéines ont été chargés sur un gel pré-coulé à 3-8% de polyacrylamide (kit Criterion XT Tris-acétate de Bio-Rad). La dystrophine a été détectée par hybridation de la membrane avec l'anticorps monoclonal primaire NCL-DYSl (Novocastra) dilué au 1:50 puis incubé avec l'anticorps secondaire anti-IgG de mouton (HRP) de mouton dilué au 1:15 000. Les protéines sont révélées par le kit SuperSignal West Pico ou Femto Maximum Sensitivity Substrate (Thermo Scientific Pierce). Les protéines musculaires totales ont été révélées en effectuant une hybridation de la membrane avec un anticorps anti-calnexine de lapin dilué au 1:2000, pendant une nuit à 4 °C, puis incubation avec de l'anticorps secondaire IgG anti-lapin (HRP) de chèvre dilué au 1:50000.

### 7/ Informations sur les cellules

### 7.1/ Tableau des patients (Tableau 8) :

| **Code Banque GNT** | **Numéro Patient** | **Type de Délétion** | **Nombre de cellules par puits** |
|---|---|---|---|
| 5-13889 | 01-001 | 48-52 | 1.8.10⁶ |
| 5-14211 | 01-009 | 52 | 1.8.10⁶ |
| 5-14476 | 01-011 | 45-52 | 1.8.10⁶ |
| 5-14496 | 01-014 | 45-52 | 1.6.10⁶ |
| 5-14878 | 01-015 | 50-52 | 1.7.10⁶ |
| 5-14498 | 01-016 | 48-52 | 1.9.10⁶ |
| 5-14499 | 01-017 | 50-52 | 1.6.10⁶ |
| 5-14589 | 01-018 | 50-52 | 1.8.10⁶ |
| 5-14683 | 01-021 | 48-52 | 1.8.10⁶ |
| 5-16283 | 02-002 | 45-52 | 1.83.10⁶ |

### 7.2/ Cellules contrôles

Les lignées cellulaires ont été fournies par la plateforme d'immortalisation cellulaire du Centre de Recherche en Myologie, de l'Université de la Sorbonne UPMC - INSERM - UMRS 974 - CNRS FRE 3617 - Institut de Myologie

### • Fibromyoblastes

Contrôle de l'expression de la dystrophine : fibroblastes humains sains J5C, myoconversion effectuée dans l'unité de recherche UMRS974.

Contrôle négatif de la restauration de la dystrophine : fibroblastes immortalisés n° F033 inéligibles (codon stop dans l'exon 16) pour le saut d'exon par JR53, myoconversion effectuée dans l'unité de recherche UMRS974.

### • Myoblastes immortalisés

Myoblastes sains pour le contrôle positif de l'expression de la dystrophine n ° 8220: hM Contrôle positif de l'efficacité du produit clinique : myoblastes DMD avec une délétion dans l'exon 52 éligibles au saut de l'exon 53, nommés KM571DMD10FL.

### B. RESULTATS

### 1/ Conversion des fibroblastes DMD humains en fibromyoblastes :

Tous les lots de fibroblastes DMD (10/10) se sont révélés positifs pour l'expression nucléaire de MyoD, montrant un pourcentage de noyaux positifs pour myoD1 par rapport au nombre total de noyaux marqués supérieur à 70% (Tableau 9), pouvant donc être considérés comme myo-convertis.

**Tableau 9: Conversion des fibroblastes DMD humains en fibromyoblastes (moyenne des pourcentages de la myoconversion pour deux expériences indépendantes).**

| **Code Banque GNT** | **Numéro Patient** | **Code Patient** | **Type de Délétion** | **Taux de Myoconversion** | **Moyenne de Myoconversion** |
|---|---|---|---|---|---|
| 5-13889 | 01-001 | BAS SA | 48-52 | 95% -100% | 98 +/- 2,5% |
| 5-14211 | 01-009 | RYF NI | 52 | 93% - 99% | 96 +/- 3% |
| 5-14476 | 01-011 | GRA IO | 45-52 | 100% - 100% | 100 +/-0% |
| 5-14496 | 01-014 | DOR AL | 45-52 | 100% - 100% | 100 +/- 0% |
| 5-14878 | 01-015 | TUD PE | 50-52 | 100% - 100% | 100 +/- 0% |
| 5-14498 | 01-016 | BEC MA | 48-52 | 68% - 90% | 79+/-11% |
| 5-14499 | 01-017 | MAR DR | 50-52 | 89% - 86% | 88 +/- 1,5% |
| 5-14589 | 01-018 | DHO RO | 50-52 | 92% - 92% | 92 +/- 0% |
| 5-14683 | 01-021 | RIC JU | 48-52 | 64% - 90% | 77 +/- 13% |
| 5-16283 | 02-002 | GRA EY | 45-52 | 79% - 86% | 83 +/- 3,5% |
| F033 DMD 16 contrôle | | | stop dans l'exon 16 | 79% - 90% | 85 +/- 5,5% |

### 2/ Efficacité de JR53 pour le saut de l'exon 53 dans les fibromyoblastes humains DMD transduits

L'efficacité de JR53 pour le saut d'exon a été testée dans deux ou trois expériences indépendantes pour chaque lot de fibromyoblastes DMD. Des produits de RT-PCR correspondant à la taille attendue en l'absence de l'exon 53 ont été détectés chez chacun des 10 patients DMD (Figure 6).

Le pourcentage d'efficacité de JR53 a été calculé à l'aide du rapport entre le nombre de pixels obtenus pour la bande sautée et la somme des pixels calculés pour l'ensemble des bandes sautées et non sautées (Tableau 10).

Pour 8 des lots de fibromyoblastes DMD, l'efficacité de JR53 a été détectée comme étant supérieure à 70%, tandis que pour les patients 5-14878 DMD et 5-14499 DMD, l'efficacité était plus faible avec une efficacité de 64 et 50%, respectivement. Les bandes correspondant à la taille de l'amplicon présentant un saut d'exon (bande inférieure) ont été séquencées et ont toutes donné la séquence attendue correspondant au saut de l'exon 53.

**Tableau 10 : Efficacité de JR53 sur le saut de l'exon 53 dans les fibromyoblastes DMD humains**

| **Code Banque GNT** | **Numéro Patient** | **Code Patient** | **Type de Délétion** | **% de saut de l'exon 53** | | | **Moyenne en % de saut de l'exon 53** | **Nombre d'expériences (n)** |
|---|---|---|---|---|---|---|---|---|
| 5-13889 | 01-001 | BAS SA | 48-52 | 100 | 83 | 100 | 94 | n=3 |
| 5-14211 | 01-009 | RYF NI | 52 | 100 | 72 | 67 | 80 | n=3 |
| 5-14476 | 01-011 | GRA IO | 45-52 | 100 | 75 | | 88 | n=2 |
| 5-14496 | 01-014 | DOR AL | 45-52 | 100 | 82 | | 91 | n=2 |
| 5-14878 | 01-015 | TUD PE | 50-52 | 60 | 72 | 60 | 64 | n=3 |
| 5-14498 | 01-016 | BEC MA | 48-52 | 100 | 84 | | 92 | n=2 |
| 5-14499 | 01-017 | MAR DR | 50-52 | **52** | **48** | | 50 | n=2 |
| 5-14589 | 01-018 | DHO RO | 50-52 | **61** | **77** | **82** | 73 | n=3 |
| 5-14683 | 01-021 | RIC JU | 48-52 | **73** | **72** | | 73 | n=2 |
| 5-16283 | 02-002 | GRA EY | 45-52 | **76** | **100** | | 88 | n=2 |

### 3/ Restauration de l'expression de la protéine dystrophine dans les fibromyoblastes DMD humains

Les résultats des westerns blots réalisés apparaissent à la figure 7.

Le niveau de restauration de l'expression de la protéine dystrophine dans les fibromyoblastes DMD humains a été évalué par la quantification du nombre de pixels correspondant au signal de chaque bande à l'aide du logiciel ImageJ. Le niveau de dystrophine a été normalisé grâce à l'expression de la calnexine (apport de protéines totales). Le pourcentage de dystrophine restaurée a été calculé à l'aide du rapport entre le taux de dystrophine normalisé dans les fibromyoblastes traités et le taux d'expression de la dystrophine observé dans les fibromyoblastes sains (hFM) considéré comme représentant 100%. Les résultats montrent que la restauration de la protéine dystrophine pouvait être observée dans tous les lots de fibromyoblastes DMD traités avec JR53. Il faut noter que pour le sujet DMD 5-14889, l'évaluation du niveau de dystrophine restaurée n'a été mesuré qu'une seule fois.

La quantification des données de restauration de l'expression de la protéine dystrophine est présentée dans le Tableau 11 :

**Tableau 11: Quantification de la restauration de la protéine dystrophine dans les fibromyoblastes DMD humains transduits**

| **Code Banque GNT** | **Numéro Patient** | **Code Patient** | **Type de Délétion** | **Restauration de la protéine dystrophine (%)** | | | **Moyenne (%)** | **Nombre d'expériences (n)** |
|---|---|---|---|---|---|---|---|---|
| 5-13889 | 01-001 | BAS SA | 48-52 | 96 | 13 | 9 | **39** | n=3 |
| 5-14211 | 01-009 | RYF NI | 52 | 18 | 28 | | **23** | n=2 |
| 5-14476 | 01-011 | GRA IO | 45-52 | 100 | 35 | | **68** | n=2 |
| 5-14496 | 01-014 | DOR AL | 45-52 | 24 | 23 | | **23,5** | n=2 |
| 5-14878 | 01-015 | TUD PE | 50-52 | 57 | 31 | 41 | **43** | n=3 |
| 5-14498 | 01-016 | BEC MA | 48-52 | 93 | 49 | | **71** | n=2 |
| 5-14499 | 01-017 | MAR DR | 50-52 | 42 | 0 | 17 | **29,5** | n=2 |
| 5-14589 | 01-018 | DHO RO | 50-52 | 6 | 0 | weak signal | **6** | n=2 |
| 5-14683 | 01-021 | RIC JU | 48-52 | 47 | 39 | | **43** | n=2 |
| 5-16283 | 02-002 | GRA EY | 45-52 | 38 | 20 | | **29** | n=2 |

### CONCLUSIONS :

L'ensemble des résultats obtenus dans le cadre de cette étude réalisés sur des fibromyoblastes humains issus de patients atteints de différentes formes de DMD sont résumés dans le Tableau 12 ci-dessous :

**Tableau 12: Résumé des données de l'efficacité de JR53 sur les fibromyoblastes DMD humains.**

| **Code Banque GNT** | **Numéro Patient** | **Code Patient** | **Type de Délétion** | **Myoconversion en %** | **Saut de l'exon 53 en %** | **Restauration de la dystrophine en %** |
|---|---|---|---|---|---|---|
| **5-13889** | 01-001 | BAS SA | 48-52 | 98 | **94** | **39** |
| **5-14211** | 01-009 | RYF NI | 52 | 96 | **80** | **23** |
| **5-14476** | 01-011 | GRA IO | 45-52 | 100 | **88** | **68** |
| **5-14496** | 01-014 | DOR AL | 45-52 | 100 | **91** | **23** |
| **5-14878** | 01-015 | TUD PDE | 50-52 | 100 | **64** | **43** |
| **5-14498** | 01-016 | BEC MA | 48-52 | 79 | **50** | **71** |
| **5-14499** | 01-017 | MAR DR | 50-52 | 88 | **50** | **29** |
| **5-14589** | 01-018 | DHO RO | 50-52 | 92 | **73** | **6** |
| **5-14683** | 01-021 | RIC JU | 48-52 | 77 | **73** | **43** |
| **5-16283** | 02-002 | GRA EY | 45-52 | 83 | **88** | **29** |

En conclusion, les résultats précliniques obtenus pour différentes formes de DMD (Δ52, Δ45-52, Δ48-52 et Δ50-52) présentés ci-dessus valident le potentiel thérapeutique de l'antisens JR53 porté par la construction testée.

## Revendications

1. Vecteur viral adéno-associé recombinant (AAVr) comprenant une séquence codant un oligonucléotide antisens (AON) dirigé contre l'intégralité de la région +30 à +69 de l'exon 53 de l'ARN pré-messager de la dystrophine, ladite séquence étant constituée de la séquence SEQ ID NO : 3.

2. Vecteur AAVr selon la revendication 1 ***caractérisé* en ce que** la séquence codant l'AON est comprise dans une séquence codant un snRNA modifié.

3. Vecteur AAVr selon la revendication 2 ***caractérisé* en ce que** le snRNA modifié est de type U7 (U7snRNA), avantageusement d'origine murine.

4. Vecteur AAVr selon la revendication 3 ***caractérisé* en ce que** le vecteur comprend la séquence SEQ ID NO : 2.

5. Vecteur AAVr selon l'une des revendications précédentes ***caractérisé* en ce que** le vecteur est choisi dans le groupe suivant : AAV2/1, AAV2, AAV2/6, AAV2/8, AAV2/9, AAV2/10, AAV8 et AAV9, avantageusement un vecteur AAV2/8.

6. Composition pharmaceutique comprenant un vecteur AAVr selon l'une des revendications 1 à 5.

7. Vecteur AAVr selon l'une des revendications 1 à 5 pour utilisation comme médicament.

8. Vecteur AAVr selon l'une des revendications 1 à 5 ou composition selon la revendication 6 pour utilisation dans le traitement de la dystrophie musculaire de Duchenne (DMD).

9. Vecteur AAVr selon l'une des revendications 1 à 5 ou composition selon la revendication 6 pour utilisation dans le traitement des formes Δ52, Δ45-52, Δ48-52 et Δ50-52 de la dystrophie musculaire de Duchenne (DMD).

## Patentansprüche

1. Rekombinanter Adeno-assoziierter viraler Vektor (AAVr), umfassend eine Sequenz, die ein Antisense-Oligonukleotid (AON) codiert, das gegen die gesamte Region von +30 bis +69 des Exons 53 der prä-Messenger-RNA von Dystrophin gerichtet ist, wobei die Sequenz aus der Sequenz SEQ ID NO: 3 besteht.

2. AAVr-Vektor gemäß Anspruch 1, **dadurch gekennzeichnet, dass** die das AON codierende Sequenz in eine Sequenz eingefügt ist, die eine modifizierte snRNA codiert.

3. AAVr-Vektor gemäß Anspruch 2, **dadurch gekennzeichnet, dass** die modifizierte snRNA vom Typ U7 (U7snRNA) und vorteilhafterweise murinen Ursprungs ist.

4. AAVr-Vektor gemäß Anspruch 3, **dadurch gekennzeichnet, dass** der Vektor die Sequenz SEQ ID NO: 2 umfasst.

5. AAVr-Vektor gemäß einem der vorhergehenden Ansprüche, **dadurch gekennzeichnet, dass** der Vektor ausgewählt ist aus der Gruppe, bestehend aus: AAV2/1, AAV2, AAV2/6, AAV2/8, AAV2/9, AAV2/10, AAV8 und AAV9, vorteilhafterweise einem AAV2/8-Vektor.

6. Pharmazeutische Zusammensetzung, umfassend einen AAVr-Vektor gemäß einem der Ansprüche 1 bis 5.

7. AAVr-Vektor gemäß einem der Ansprüche 1 bis 5 zur Verwendung als Medikament.

8. AAVr-Vektor gemäß einem der Ansprüche 1 bis 5 oder die Zusammensetzung gemäß Anspruch 6 zur Verwendung bei der Behandlung von Muskeldystrophie Duchenne (DMD).

9. AAVr-Vektor gemäß einem der Ansprüche 1 bis 5 oder die Zusammensetzung gemäß Anspruch 6 zur Verwendung bei der Behandlung der Formen Δ52, *Δ*45-52, *Δ*48-52 und *Δ*50-52 von Muskeldystrophie Duchenne (DMD).

## Claims

1. A recombinant adeno-associated viral vector (AAVr) comprising a sequence coding an antisense oligonucleotide (AON) directed against the entire +30 to +69 region of exon 53 of the pre-messenger RNA of dystrophin, said sequence consisting of the sequence SEQ ID NO: 3.

2. The AAVr vector according to claim 1, ***characterized in that*** the sequence encoding the AON is inserted in a sequence coding a modified snRNA.

3. The AAVr vector according to claim 2, ***characterized in that*** the modified snRNA is of type U7 (U7snRNA), advantageously of murine origin.

4. The AAVr vector according to claim 3, ***characterized in that*** the vector comprises the sequence SEQ ID NO: 2.

5. The AAVr vector according to one of the preceding claims, ***characterized in that*** the vector is selected from the group consisting of : AAV2/1, AAV2, AAV2/6, AAV2/8, AAV2/9, AAV2/10, AAV8 and AAV9, advantageously an AAV2/8 vector.

6. A pharmaceutical composition comprising an AAVr vector according to one of claims 1 to 5.

7. The AAVr vector according to one of claims 1 to 5 for use as a medicament.

8. The AAVr vector according to one of claims 1 to 5 or the composition according to claim 6 for use in the treatment of Duchenne muscular dystrophy (DMD).

9. The AAVr vector according to one of claims 1 to 5 or the composition according to claim 6 for use in the treatment of forms Δ52, Δ45-52, Δ48-52 and Δ50-52 of Duchenne muscular dystrophy (DMD).
